# EUROPEAN PATENT SPECIFICATION

(11) **EP 1 343 765 B1**
(45) Date of publication and mention of the grant of the patent: **22.11.2006**
(21) Application number: 01271361.6
(22) Date of filing: 19.12.2001
(51) Int. Cl.: C07D 215/20, C07D 401/12, C07D 409/12, C07D 407/12, C07D 413/12, A61K 31/33, A61P 31/04

(54) **QUINOLINES AND NITROGENATED DERIVATIVES THEREOF SUBSTITUTED IN 4-POSITION BY A PIPERAZINE-CONTAINING MOIETY AND THEIR USE AS ANTIBACTERIAL AGENTS**
CHINOLINE UND DEREN IN DER 4-STELLUNG DURCH EINE PIPERAZINHALTIGE GRUPPE SUBSTITUIERTE NITROGENIERTE DERIVATE UND DEREN VERWENDUNG ALS ANTIBAKTERIELLE MITTEL
QUINOLEINES ET DERIVES AZOTES DE CELLES-CI SUBSTITUES A LA POSITION 4 PAR UN FRAGMENT CONTENANT DE LA PIPERAZINE ET UTILISATION DE CEUX-CI EN TANT QU'AGENTS ANTIBACTERIENS

(30) Priority: 20.12.2000 GB 0031086
(43) Date of publication of application: 17.09.2003
(73) Proprietor: SMITHKLINE BEECHAM PLC, Brentford, Middlesex TW8 9GS (GB)
(72) Inventor: MARKWELL, Roger, Edward, c/o GlaxoSmithKline, Harlow, Essex CM19 5AW (GB); PEARSON, Neil, David, GlaxoSmithKline, Harlow, Essex CM19 5AW (GB); SMETHURST, Christian, GlaxoSmithKline, Harlow, Essex CM19 5AW (GB)
(74) Representative: Valentine, Jill Barbara
(86) International application number: PCT/GB2001/005661
(87) International publication number: WO 2002/050040

(56) References cited:
- WO-A-00/21948
- WO-A-00/34265
- WO-A-00/43383
- WO-A-00/78748
- WO-A-99/37635
- FR-A- 2 798 656
- R. RASTOGI ET AL.: "Synthesis of N-(2-Substituted-ethyl)-N'-arylpiperazines and their Quaternary Salts as potential Antihookworm Agents" INDIAN J. CHEM. SECT. B, vol. 19B, 1980, pages 1003-1005, XP001062580
- KAYIRERE M-G ET AL: "Synthesis and antibacterial activity of new 4-alkoxy, 4-aminoalkyl and 4-alkylthioquinoline derivatives" EUROPEAN JOURNAL OF MEDICINAL CHEMISTRY, EDITIONS SCIENTIFIQUE ELSEVIER, PARIS, FR, vol. 33, no. 1, January 1998 (1998-01), pages 55-63, XP004173056 ISSN: 0223-5234
- PATENT ABSTRACTS OF JAPAN vol. 1996, no. 08, 30 August 1996 (1996-08-30) & JP 08 092219 A (POLA CHEM IND INC), 9 April 1996 (1996-04-09)

## Description

This invention relates to novel compounds, compositions containing them and their use as antibacterials.

WO99/37635, WO00/21948 and WO00/21952 disclose piperidine derivatives having antibacterial activity.

EP0579263, EP0742207, JP2169569 and EP0296560, generically disclose piperazine compounds as acetylcholinesterase inhibitors and sigma receptor antagonists

WO92/17475, WO98/02438, WO97/03069 and WO96/39145 disclose certain bicyclic heteroaromatic compounds having cholinesterase inhibitor, protein tyrosine kinase inhibitor, cell proliferation inhibitor and human epidermal growth factor receptor type 2 inhibitor activity.

JP7179407 discloses bicyclic heteroaromatic compounds having GPIIb/IIIa inhibitory activity

WO97/17973 discloses piperazine derivatives having hemoregulatory activities.

WO99/05096 discloses naphthamidine compounds having urokinase inhibitory activity.

This invention provides a compound of formula (I) or a pharmaceutically acceptable acid addition salt, quaternary ammonium salt, solvate and/or N-oxide thereof: 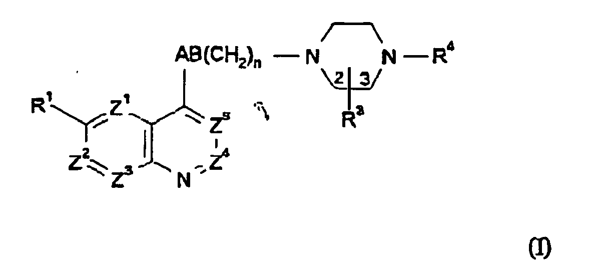 wherein:
one of Z¹, Z², Z³, Z⁴ and Z⁵ is N, one is CR^{1a} and the remainder are CH, or one of Z¹, Z², Z³, Z⁴ and Z⁵ is CR^{1a} and the remainder are CH;
or when Z⁵ is CR^{1a}, R^{1a} may instead be cyano, hydroxymethyl or carboxy;
R¹ and R^{1a} are independently selected from hydrogen; hydroxy; (C₁₋₆) alkoxy optionally substituted by (C₁₋₆)alkoxy, amino, piperidyl, guanidino or amidino any of which is optionally N-substituted by one or two (C₁₋₆)alkyl, acyl or (C₁₋₆)alkylsulphonyl groups, (C₁₋₆)alkylthio, heterocyclylthio, heterocycl yloxy, arylthio, aryloxy, acylthio, acyloxy or (C₁₋₆)alkylsulphonyloxy; (C₁₋₆)alkoxy-suhstituted (C₁₋ 6)alkyl; halogen; (C₁₋₆)alkyl; (C₁₋₆)alkylthio; trifluoromethyl; nitro; azido; acyl; acyloxy; acylthio; (C₁₋₆)alkylsulphonyl; (C₁₋₆)alkylsulphoxide; arylsulphonyl; arylsulphoxide or an amino, piperidyl, guanidino or amidino group optionally N-substituted by one or two (C₁₋₆)alkyl, acyl or (C₁₋₆)alkylsulphonyl groups, provided that when none of Z¹, Z², Z³, Z⁴ and Z⁵ is N, then R¹ is not hydrogen;
R³ is hydrogen; or
R³ is in the 2- or 3-position and is: carboxy; (C₁₋₆)alkoxycarbonyl; aminocarbonyl wherein the amino group is optionally substituted by hydroxy, (C₁₋₆)alkyl, hydroxy(C₁₋₆)alkyl, aminocarbonyl(C₁₋₆)alkyl, (C₂₋₆)alkenyl, (C₁₋₆)alkylsulphonyl, trifluoromethylsulphonyl, (C₂₋₆)alkenylsulphonyl, (C₁₋₆)alkoxycarbonyl, (C ₁₋₆)alkylcarbonyl, (C₂₋₆)alkenyloxycarbonyl or (C₂₋₆)alkenylcarbonyl and optionally further substituted by (C₁₋₆)alkyl, hydroxy(C₁₋₆)alkyl, aminocarbonyl(C₁₋₆)alkyl or (C₂₋₆)alkenyl; cyano; tetrazolyl; 2-oxo-oxazolidinyl optionally substituted by R¹⁰; 3-hydroxy-3-cyclobutene-1,2-dione-4-yl; 2,4-thiazolidinedione-5-yl; tetrazol-5-ylaminocarbonyl; 1,2,4-triazol-5-yl optionally substituted by R¹⁰; or 5-oxo-1,2,4-oxadiazol-3-yl; or
   (C₁₋₄)alkyl or ethenyl optionally substituted with any of the groups listed above for R³ and/or 0 to 2 groups R¹² independently selected from:
   halogen; (C₁₋₆)alkylthio; trifluoromethyl; (C₁₋₆)alkoxycarbonyl; (C₁₋₆)alkylcarbonyl; (C₂₋₆)alkenyloxycarbonyl; (C₂₋₆)alkenylcarbonyl; hydroxy optionally substituted by (C₁₋₆)alkyl, (C₂₋₆)alkenyl, (C₁₋₆)alkoxycarbonyl, (C₁₋₆)alkylcarbonyl, (C₂₋₆)alkenyloxycarbonyl, (C₂₋₆)alkenylcarbonyl or aminocarbonyl wherein the amino group is optionally substituted by (C₁₋₆)alkyl, (C₂₋₆)alkenyl, (C₁₋₆)alkylcarbonyl or (C₂₋₆)alkenylcarbonyl; amino optionally mono- or disubstituted by (C₁₋₆)alkoxycarbonyl, (C₁₋₆)alkylcarbonyl, (C₂₋₆)alkenyloxycarbonyl, (C₂₋₆)alkenylcarbonyl, (C ₁₋₆)alkyl, (C₂₋₆)alkenyl, (C₁₋₆)alkylsulphonyl, (C₂₋₆)alkenylsulphonyl or aminocarbonyl wherein the amino group is optionally substituted by (C₁₋₆)alkyl or (C₂₋₆)alkenyl; aminocarbonyl wherein the amino group is optionally substituted by (C₁₋₆)alkyl, hydroxy(C₁₋₆)alkyl, aminocarbonyl(C₁₋₆)alkyl, (C₂₋₆)alkenyl, (C₁₋₆)alkoxycarbonyl, (C₁₋₆)alkylcarbonyl, (C₂₋₆)alkenyloxycarbonyl or (C₂₋₆)alkenylcarbonyl and optionally further substituted by (C₁₋₆)alkyl, hydroxy(C₁₋₆)alkyl, aminocarbonyl(C₁₋₆)alkyl or (C₂₋₆)alkenyl; oxo; (C₁₋₆)alkylsulphonyl; (C₂₋₆)alkenylsulphonyl; or (C₁₋₆)aminosulphonyl wherein the amino group is optionally substituted by (C₁₋₆)alkyl or (C₂₋₆)alkenyl; in addition when R³ is disubstituted with a hydroxy or amino containing substituent and a carboxy containing substituent these may optionally together form a cyclic ester or amide linkage, respectively;
R¹⁰ is selected from (C₁₋₄)alkyl; (C₂₋₄)alkenyl and aryl any of which may be optionally substituted by a group R¹² as defined above; carboxy; aminocarbonyl wherein the amino group is optionally substituted by hydroxy, (C₁₋₆)alkyl, (C₂₋₆)alkenyl, (C₁₋₆)alkylsulphonyl, trifluoromethylsulphonyl, (C₂₋₆)alkenylsulphonyl, (C_{1 6})alkoxycarbonyl, (C₁₋₆)alkylcarbonyl, (C₂₋₆)alkenyloxycarbonyl or (C₂₋₆)alkenylcarbonyl; and
R⁴ is a group -V-X¹-X²-X³-X⁴ in which:
   V is CH₂, CO or SO₂;
   X¹ is CR¹⁴R¹⁵;
   X² is NR¹³, O, SO₂ or CR¹⁴R¹⁵;
   X³ is NR¹³, O or CR¹⁴R¹⁵; wherein:
      each of R¹⁴ and R¹⁵ is independently selected from: hydrogen; (C₁₋₄)alkoxy; (C₁₋₄)alkylthio; trifluoromethyl; cyano; (C₁₋₄)alkyl; (C₂₋₄)alkenyl; (C₁₋₄)alkoxycarbonyl; (C₁₋₄)alkylcarbonyl; (C₂₋₄)alkenyloxycarbonyl; (C₂₋₄)alkenylcarbonyl; hydroxy, amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C₁₋₄)alkyl or (C₂₋₄)alkenyl, provided that R¹⁴ and R¹⁵ on the same carbon atom are not both selected from optionally substituted hydroxy and optionally substituted amino; or
   R¹⁴ and R¹⁵ together represent oxo;
   R¹³ is hydrogen; trifluoromethyl, (C₁₋₆)alkyl; (C₂₋₆)alkenyl; (C₁₋₆)alkoxycarbonyl; (C₁₋₆)alkylcarbonyl; aminocarbonyl wherein the amino group is optionally substituted by (C₁₋₆)alkoxycarbonyl, (C₁₋₆)alkylcarbonyl, (C₂₋₆)alkenyloxycarbonyl, (C₂₋₆)alkenylcarbonyl, (C₁₋₆)alkyl or (C₂₋₆)alkenyl and optionally further substituted by (C₁₋₆)alkyl or (C₂₋₆)alkenyl;
   two R¹⁴ groups or an R¹³ and an R¹⁴ group in X¹ ,X² and X³ together with the atoms to which they are attached and, if appropriate, the intervening group X² form a 5 or 6 membered carbocyclic or heterocyclic ring and the remaining R¹³, R¹⁴ and R¹⁵ groups are as above defined; or
   two R¹⁴ groups or an R¹³ and an R¹⁴ group on adjacent atoms together represent a bond and the remaining R¹³, R¹⁴ and R¹⁵ groups are as above defined;
   X⁴ is phenyl or C or N linked monocyclic aromatic 5- or 6-membered heterocycle containing up to four heteroatoms selected from O, S and N and optionally C-substituted by up to three groups selected from (C₁₋₄)alkylthio; halo; carboxy(C₁₋₄)alkyl; halo(C₁₋₄)alkoxy; halo(C₁₋₄)alkyl; (C₁₋₄)alkyl; (C₂₋₄)alkenyl; (C₁₋₄)alkoxycarbonyl; formyl; (C₁₋₄)alkylcarbonyl; (C₂₋₄)alkenyloxycarbonyl; (C₂₋₄)alkenylcarbonyl; (C₁₋₄)alkylcarbonyloxy; (C₁₋₄)alkoxycarbonyl(C₁₋₄)alkyl; hydroxy; hydroxy(C₁₋₄)alkyl; mercapto(C₁₋₄)alkyl; (C₁₋₄)alkoxy; nitro; cyano; carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C₁₋₄)alkyl or (C₂₋₄)alkenyl; aryl; aryl(C₁₋₄)alkyl or aryl(C₁₋₄)alkoxy; and optionally N substituted by trifluoromethyl; (C₁₋₄)alkyl optionally substituted by hydroxy, (C₁₋₆)alkoxy, (C₁₋₆)alkylthio, halo or trifluoromethyl; (C₂₋₄)alkenyl; aryl; aryl(C₁₋₄)alkyl; (C₁₋₄)alkoxycarbonyl; (C₁₋₄)alkylcarbonyl; formyl; (C₁₋₆)alkylsulphonyl; or aminocarbonyl wherein the amino group is optionally substituted by (C₁₋₄)alkoxycarbonyl, (C₁₋₄)alkylcarbonyl, (C₂₋₄)alkenyloxycarbonyl,(C₂₋₄)alkenylcarbonyl, (C₁₋₄)alkyl or (C₂₋₄)alkenyl and optionally further substituted by (C₁₋₄)alkyl or (C₂₋₄)alkenyl;
   provided that when X⁴ is optionally substituted phenyl, -V-X ¹-X²-X³- is not -(CH₂)₃O-, -CH₂-(C₃-alkenyl)- or -(CH₂)₃CO-;
   n is 0 and AB is NR¹¹CO, CO-CR⁸R⁹, CR⁶R⁷-CO, NR¹¹SO₂, CR⁶R⁷-SO₂ or CR⁶R⁷⁻CR⁸R⁹, provided that R⁸ and R⁹ are not optionally substituted hydroxy or amino and R⁶ and R⁸ do not represent a bond:
   or n is 1 and AB is NR¹¹CO, CO-CR⁸R⁹, CR⁶R⁷-CO, NR¹¹SO₂, CONR¹¹, CR⁶R⁷⁻CR⁸R⁹, O-CR⁸R⁹ or NR¹¹-CR⁸R⁹;
   and wherein:
      each of R⁶ and R⁷, R⁸ and R⁹ is independently selected from: H; (C₁₋₆)alkoxy; (C₁₋₆)alkylthio; halo; trifluoromethyl; azido; (C₁₋₆)alkyl; (C₂₋₆)alkenyl; (C₁₋₆)alkoxycarbonyl; (C₁₋₆)alkylcarbonyl; (C₂₋₆)alkenyloxycarbonyl; (C₂₋₆)alkenylcarbonyl; hydroxy, amino or aminocarbonyl optionally substituted as for corresponding substituents in R³; (C₁₋₆)alkylsulphonyl; (C₂₋₆)alkenylsulphonyl; or (C₁₋₆)aminosulphonyl wherein the amino group is optionally substituted by (C ₁₋₆)alkyl or (C₂₋₆)alkenyl;
      or R⁶ and R⁸ together represent a bond and R⁷ and R⁹ are as above defined;
   and each R¹¹ is independently H; trifluoromethyl; (C₁₋₆)alkyl; (C₂₋₆)alkenyl; (C₁₋₆)alkoxycarbonyl; (C₁₋₆)alkylcarbonyl; or aminocarbonyl wherein the amino group is optionally substituted by (C₁₋₆)alkoxycarbonyl, (C₁₋₆)alkylcarbonyl, (C₂₋₆)alkenyloxycarbonyl, (C₂₋₆)alkenylcarbonyl, (C₁₋₆)alkyl or (C₂₋₆)alkenyl and optionally further substituted by (C₁₋₆)alkyl or (C₂₋₆)alkenyl;
   or where one of R³ and R⁶, R⁷, R⁸ or R⁹ contains a carboxy group and the other contains a hydroxy or amino group they may together form a cyclic ester or amide linkage.

The invention also provides the use of a compound of formula (I) or a pharmaceutically acceptable acid addition salt, quaternary ammonium salt, solvate and/or N-oxide thereof in the manufacture of a medicament for use in the treatment of bacterial infections in mammals.

The invention also provides a pharmaceutical composition for use in the treatment of bacterial infections in mammals comprising a compound of formula (I), or a pharmaceutically acceptable acid addition salt, quaternary ammonium salt, solvate and/or N-oxide thereof, and a pharmaceutically acceptable carrier.

A method of treatment of bacterial infections in mammals, particularly in man, comprises the administration to a mammal in need of such treatment of an effective amount of a a compound of formula (I), or a pharmaceutically acceptable acid addition salt, quaternary ammonium salt, solvate and/or N-oxide thereof.

In one aspect, when Z⁵ is N, AB(CH₂)ₙ is (CH₂)₂, (CH₂)₃, CH=CHCH₂, NR¹¹(CH₂)₂ or O(CH₂)₂ and -V-X¹-X²-X³- is (CH₂)₄, X⁴ is not optionally substituted phenyl or an optionally substituted monocyclic aromatic 5-membered heterocycle.

Preferably Z⁵ is CH or N, Z³ is CH or CF and Z¹, Z² and Z⁴ are each CH, or Z¹ is N, Z³ is CH or CF and Z², Z⁴ and Z⁵ are each CH.

When R¹ or R^{1a} is substituted alkoxy it is preferably (C₂₋₆)alkoxy substitituted by optionally N-substituted amino, guanidino or amidino, or (C₁₋₆)alkoxy substituted by piperidyl. Suitable examples of R¹ alkoxy include methoxy, n-propyloxy, i-butyloxy, aminoethyloxy, aminopropyloxy, aminobutyloxy, aminopentyloxy, guanidinopropyloxy, piperidin-4-ylmethyloxy, phthalimido pentyloxy or 2-aminocarbonylprop-2-oxy.

Preferably R¹ is methoxy, amino(C₃₋₅)alkyloxy, guanidino(C₃₋₅)alkyloxy, piperidyl(C₃₋₅)alkyloxy, nitro or fluoro; more preferably methoxy, amino(C₃₋₅)alkyloxy or guanidino(C₃₋₅)alkyloxy. Preferably R^{1a} is H or F. Most preferably R¹ is methoxy and R^{1a} is H or when Z³ is CR^{1a} it may be C-F.

When Z⁵ is CR^{1a}, R^{1a} is preferably hydrogen, cyano, hydroxymethyl or carboxy, most preferably hydrogen.

Preferably n is 0.

Preferred examples of R³ include hydrogen; (C₁₋₄) alkyl; ethenyl; optionally substituted 1-hydroxy-(C₁₋₄) alkyl; optionally substituted aminocarbonyl; carboxy(C₁₋₄)alkyl; optionally substituted aminocarbonyl(C₁₋₄)alkyl; cyano(C₁₋₄)alkyl; optionally substituted 2-oxo-oxazolidinyl and optionally substituted 2-oxo-oxazolidinyl(C₁₋₄alkyl). More preferred R³ groups are hydrogen; CONH₂; 1-hydroxyalkyl e.g. CH₂OH, CH(OH)CH₂CN; CH₂CO₂H; CH₂CONH₂; -CONHCH₂CONH₂; 1,2-dihydroxyalkyl e.g. CH(OH)CH₂OH; CH₂CN; 2-oxo-oxazolidin-5-yl and 2-oxo-oxazohdin-5-yl(C₁₋₄alkyl). Most preferably R³ is hydrogen.

When R³ and R⁶, R⁷, R⁸ or R⁹ together form a cyclic ester or amide linkage, it is preferred that the resulting ring is 5-7 membered. It is further preferred that the group A or B which does not form the ester or amide linkage is CH₂.

When A is CH(OH) the R-stereochemistry is preferred.

Preferably A is NH, NCH₃, CH₂, CHOH, CH(NH₂), C(Me)(OH) or CH(Me).

Preferably B is CH₂ or CO.

Preferably n=0.

Most preferably:
n is 0 and either A is CHOH and B is CH₂ or A is NH and B is CO.

Preferably R¹¹ is hydrogen or (C₁₋₄)alkyl e.g. methyl, more preferably hydrogen.

V is preferably CH₂.

R¹³, R¹⁴ and R¹⁵ are preferably H or (C₁₋₄)alkyl such as methyl, more preferably hydrogen.

X¹ is preferably CH₂.

X² is preferably CH₂ or together with X³ forms a CH=CH group.

X³ is preferably CH₂, O or NH, or together with X² forms a CH=CH group.

Preferred linker groups -X¹-X²-X³- include -(CH₂)₂-O-, -CH₂-CH=CH-, - (CH₂)₃-, -(CH₂)₂-NH-, -CH(OH) -CH₂-NH-, -CH₂NHCO- or -CH₂CONH-. Where the linker group is a cyclised groups, two R¹⁴ groups or R¹³ and R¹⁴ complete a carbocylic or heterocyclic ring such as oxazolidin-2-one. The cyclised linker group - X¹-X²-X³-is preferably oxazolidin-2-one-3,5-diyl where X³ is N.

Monocyclic aromatic heterocyclic groups for X⁴ include pyridyl, pyrazinyl, pyrimidinyl, triazolyl, tetrazolyl, thienyl, isoimidazolyl, thiazolyl, furanyl and imidazolyl, all preferably C-linked, and N-linked 2H-pyridazone and 1H-pyrid-2-one. Preferred aromatic heterocyclic groups include pyrid-2-yl, pyrid-3-yl, 1,3-thiazole-2-yl, pyrimidin-2-yl, pyrimidin-5-yl and fur-2-yl.

Heterocyclic X⁴ is preferably unsubstituted or substituted by a group selected from halo especially fluoro, trifluoromethyl and nitro, most preferably substituted by fluoro.

Preferred substituents on phenyl X⁴ include halo, especially fluoro, nitro, cyano, trifluoromethyl, methyl, methoxycarbonyl and methylcarbonylamino.

Preferably X⁴ is 2-pyridyl, 3-fluorophcnyl, 3,5-difluorophenyl or 1,3-thiazole-2-yl.

When used herein, the term "alkyl" includes groups having straight and branched chains, for instance, methyl, ethyl, n-propyl, iso-propyl, n-butyl, iso-butyl, sec-butyl, t-butyl, pentyl and hexyl. The term 'alkenyl' should be interpreted accordingly.

Halo or halogen includes fluoro, chloro, bromo and iodo.

Haloalkyl moieties include 1-3 halogen atoms.

Unless otherwise defined, the term 'heterocyclic' as used herein includes aromatic and non-aromatic, single and fused, rings suitably containing up to four hetero-atoms in each ring selected from oxygen, nitrogen and sulphur, which rings may be unsubstituted or C-substituted by, for example, up to three groups selected from (C₁₋₄)alkylthio; halo; carboxy(C₁₋₄)alkyl; halo(C₁₋₄)alkoxy; halo(C₁₋₄)alkyl; (C₁₋₄)alkyl; (C₂₋₄)alkenyl; (C₁₋₄)alkoxycarbonyl; formyl; (C₁₋₄)alkylcarbonyl; (C₂₋₄)alkenyloxycarbonyl; (C₂₋₄)alkenylcarbonyl; (C₁₋₄)alkylcarbonyloxy; (C₁₋₄)alkoxycarbonyl(C₁₋₄)alkyl; hydroxy; hydroxy(C₁₋₄)alkyl; mercapto(C₁₋₄)alkyl; (C₁₋₄)alkoxy; nitro; cyano; carboxy; amino or aminocarbonyl optionally substituted as for corresponding substituents in R³; (C₁₋₄)allcylsulphonyl; (C₂₋₄)alkenylsulphonyl; or aminosulphonyl wherein the amino group is optionally substituted by (C₁₋₄)alkyl or (C₂₋₄)alkenyl; optionally substituted aryl, aryl(C₁₋₄)alkyl or aryl(C₁₋₄)alkoxy and oxo groups. Each heterocyclic ring suitably has from 4 to 7, preferably 5 or 6, ring atoms. A fused heterocyclic ring system may include carbocyclic rings and need include only one heterocyclic ring. Compounds within the invention containing a heterocyclyl group may occur in two or more tautometric forms depending on the nature of the heterocyclyl group; all such tautomeric forms are included within the scope of the invention.

Where an amino group forms part of a single or fused non-aromatic heterocyclic ring as defined above suitable optional substituents in such substituted amino groups include H; trifluoromethyl; (C₁₋₄)alkyl optionally substituted by hydroxy, (C₁₋₆)alkoxy, (C₁₋₆)alkylthio, halo or trifluoromethyl; (C₂₋₄)alkenyl; aryl; aryl (C₁₋₄)alkyl; (C₁₋₄)alkoxycarbonyl; (C₁₋₄)alkylcarbonyl; formyl; (C₁₋₆)alkylsulphonyl; or aminocarbonyl wherein the amino group is optionally substituted by (C₁₋₄)alkoxycarbonyl, (C₁₋₄)alkylcarbonyl, (C₂₋₄)alkenyloxycarbonyl (C₂₋₄)alkenylcarbonyl, (C₁₋₄)alkyl or (C₂₋₄)alkenyl and optionally further substituted by (C₁₋₄)alkyl or (C₂₋₄)alkenyl.

When used herein the term 'aryl', includes phenyl and naphthyl, each optionally substituted with up to five, preferably up to three, groups selected from(C₁₋₄)alkylthio; halo; carboxy(C₁₋₄)alkyl; halo(C₁₋₄)alkoxy, halo(C₁₋₄)alkyl; (C₁₋₄)alkyl; (C₂₋₄)alkenyl; (C₁₋₄)alkoxycarbonyl; formyl; (C₁₋₄)alkylcarbonyl; (C₂₋₄)alkenyloxycarbonyl; (C₂₋₄)alkenylcarbonyl; (C₁₋₄)alkylcarbonyloxy; (C₁₋₄)alkoxycarbonyl(C₁₋₄)alkyl; hydroxy; hydroxy(C₁₋₄)alkyl; mercapto(C₁₋₄)alkyl; (C₁₋₄)alkoxy; nitro; cyano, carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C₁₋₄)alkyl or (C₂₋₄)alkenyl; phenyl, phenyl(C₁₋₄)alkyl or phenyl(C₁₋₄)alkoxy.

The term 'acyl' includes (C₁₋₆)alkoxycarbonyl, formyl or (C₁₋₆) alkylcarbonyl groups.

Preferred compounds of formula (I) include:
3 3-(3,5-Difluoro-phenyl)-5-{4-[*(R)*-2-hydroxy-2-(6-methoxy-quinolin-4-yl)-ethyl]-piperazin-1-ylmethyl}-oxazolidin-2-one
3,5-Difluoro-*N*-(2-{4-[*(R)*-2-hydroxy-2-(6-methoxy-quinolin-4-yl)-ethyl]-piperazin-1-yl}-ethyl)-benzamide
*(R)*-1-(6-Methoxy-quinolin-4-yl)-2-[4-(4-phenyl-butyl)-piperazin-1-yl]-ethanol
*N*-(3,5-Difluoro-phenyl)-3-{4-[*(R)*-2-hydroxy-2-(6-methoxy-quinolin-4-yl)-ethyl]-piperazin-1-yl}-propionamide
3-(3-Fluoro-phenyl)-5-{4-[*(R)*-2-hydroxy-2-(6-methoxy-quinolin-4-yl)-ethyl]-piperazin-1-ylmethyl}-oxazolidin-2-one
and pharmaceutically acceptable acid addition salts, quaternary ammonium salts, solvates and/or N-oxide thereof.

Some of the compounds of this invention may be crystallised or recrystallised from solvents such as organic solvents. In such cases solvates may be formed. This invention includes within its scope stoichiometric solvates including hydrates as well as compounds containing variable amounts of water that may be produced by processes such as lyophilisation.

Since the compounds of formula (I) are intended for use in pharmaceutical compositions it will readily be understood that they are each provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions; these less pure preparations of the compounds should contain at least 1%, more suitably at least 5% and preferably from 10 to 59% of a compound of the formula (I) or pharmaceutically acceptable derivative thereof.

Pharmaceutically acceptable derivatives of the above-mentioned compounds of formula (I) include the free base form or their acid addition or quaternary ammonium salts, for example their salts with mineral acids e.g. hydrochloric, hydrobromic, sulphuric nitric or phosphoric acids, or organic acids, e.g. acetic, fumaric, succinic, maleic, citric, benzoic, p-toluenesulphonic, methanesulphonic, naphthalenesulphonic acid or tartaric acids. Compounds of formula (I) may also be prepared as the N-oxide. The invention extends to all such derivatives.

Certain of the above-mentioned compounds of formula (I) may exist in the form of optical isomers, e.g. diastereoisomers and mixtures of isomers in all ratios, e.g. racemic mixtures. The invention includes all such forms, in particular the pure isomeric forms. For examples the invention includes compound in which an A-B group CH(OH)-CH₂ is in either isomeric configuration the *R*-isomer is preferred. The different isomeric forms may be separated or resolved one from the other by conventional methods, or any given isomer may be obtained by conventional synthetic methods or by stereospecific or asymmetric syntheses.

In a further aspect of the invention there is provided a process for preparing compounds of formula (I), or a pharmaceutically acceptable acid addition salt, quaternary ammonium salt, solvate and/or N-oxide thereof, which process comprises reacting a compound of formula (IV) with a compound of formula (V): 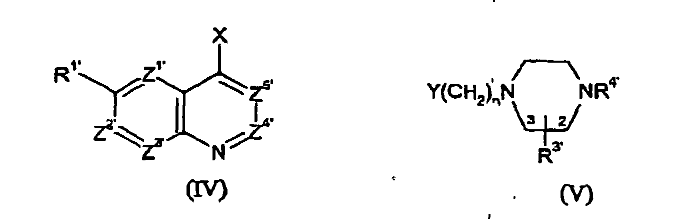 wherein n is as defined in formula (I); Z^{1'}, Z^{2'}, Z^{3'}, Z^{4'}, Z^{5'}, R^{1'}, R^{3'} and R^{4'} are Z¹, Z², Z³, Z⁴, Z⁵, R¹, R³ and R⁴ as defined in formula (I) or groups convertible thereto; and X and Y may be the following combinations:
(i) X is A'-COW, Y is H and n is 0;
(ii) X is CR⁶=CR⁸R⁹, Y is H and n is 0;
(iii) X is oxirane, Y is H and n is 0;
(iv) X is N=C=O and Y is H and n is 0;
(v) one of X and Y is CO₂Ry and the other is CH₂CO₂R^{x};
(vi) X is CHR⁶R⁷ and Y is C(=O)R⁸;
(vii) X is CR⁷=PR^{z}₃ and Y is C(=O)R⁹ and n=1;
(viii) X is C(=O)R⁷ and Y is CR⁹=PR^{z}₃ and n=1;
(ix) Y is COW and X is NHR^{11'} or NR^{11'}COW and n=0 or 1 or when n=1 X is COW and Y is NHR^{11'}, NCO or NR^{11'}COW;
(x) X is NHR^{11'} and Y is C(=O)R⁸ and n=1;
(xi) X is NHR^{11'} and Y is CR⁸R⁹W and n=1;
(xii) X is NR^{11'}COCH₂W or NR^{11'}SO₂CH₂W and Y is H and n=0;
(xiii) X is CR⁶R⁷SO₂W and Y is H and n=0;
(xiv) X is W or OH and Y is CH₂OH and n is 1;
(xv) X is NHR^{11'} and Y is SO₂W or X is NR^{11'}SO₂W and Y is H, and n is 0;
in which W is a leaving group, e.g. halo or imidazolyl; R^{x} and R^{y} are (C₁₋₆)alkyl; R^{z} is aryl or (C₁₋₆)alkyl; A' and NR^{11'} are A and NR¹¹ as defined in formula (I), or groups convertible thereto; and oxirane is: 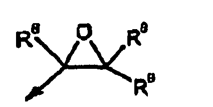 wherein R⁶, R⁸ and R⁹ are as defined in formula (I); and thereafter optionally or as necessary converting A', Z^{1'}, Z^{2'}, Z^{3'},Z^{4'}, Z^{5'}, R^{1'}, R^{3'}, R^{4'} and NR^{11'} to A, Z¹, Z², Z³, Z⁴, Z⁵, R¹, R³, R⁴ and NR¹¹, converting A-B to other A-B, interconverting R¹, R³ and/or R⁴, and/or forming a pharmaceutically acceptable acid addition salt, quaternary ammonium salt, solvate and/or N-oxide thereof.

Process variant (i) initially produces compounds of formula (I) wherein A-B is A'-CO.

Process variant (ii) initially produces compounds of formula (I) wherein A-B is CHR⁶-CR⁸R⁹.

Process variant (iii) initially produces compounds of formula (I) wherein A-B is CR⁶(OH)-CR⁸R⁹.

process variant (iv) initially produces compounds of formula (I) where A-B is NH-CO.

Process variant (v) initially produces compounds of formula (I) wherein A-B is CO-CH₂ or CH₂-CO.

process variant (vi) initially produces compounds of formula (I) wherein A-B is CR⁶R⁷-CR⁸OH.

Process variant (vii) and (viii) initially produce compounds of formula (I) wherein A-B is CR⁶=CR⁸.

Process variant (ix) initially produces compounds of formula (I) where A-B is CO-NR¹¹ or NR¹¹-CO.

Process variant (x) initially produces compounds of formula (I) wherein A-B is NR¹¹-CHR⁸.

Process variant (xi) initially produces compounds of formula (I) wherein A-B is NR^{11'}-CR⁸R⁹.

process variant (xii) initially produces compounds of formula (I) where A-B is NR^{11'}-CO or NR^{11'}SO₂ and n=1.

Process variant (xiii) initially produces compounds of formula (I) where A-B is CR⁶R⁷-SO₂.

Process variant (xiv) initially produces compounds of formula (I) wherein A-B is O-CH₂.

Process variant (xv) initially produces compounds where AB is NR¹¹SO₂.

In process variants (i) and (ix) the reaction is a standard amide or urea formation reaction involving e.g.:
1. Activation of a carboxylic acid (e.g. to an acid chloride, mixed anhydride, active ester, O-acyl-isourea or other species), and treatment with an amine (Ogliaruso, M.A.; Wolfe, J.F. in *The Chemistry of Functional Groups (Ed. Patai, S.) Suppl. B: The Chemistry of Acid Derivatives, Pt. 1* (John Wiley and Sons, 1979), pp 442-8; Beckwith, A.L.J., in *The Chemistry of Functional Groups (Ed. Patai, S.) Suppl. B: The Chemistry of Amides (Ed. Zabricky, J.)* (John Wiley and Sons, 1970), p 73 ff. The acid and amine are preferably reacted in the presence of an activating agent such as 1-(dimethylaminopropyl)-3-ethylcarbodiimide hydrochloride (EDC) or 1-hydroxybenzotriazole (HOBT); or
2. The specific methods of:
   a. *in situ* conversion of an acid into the amine component by a modified Curtius reaction procedure (Shioiri, T., Murata, M., Hamada, Y., *Chem. Pharm. Bull.* 1987, 35, 2698)
   b. *in situ* conversion of the acid component into the acid chloride under neutral conditions (Villeneuve, G. B.; Chan, T. H., *Tetrahedron. Lett.* 1997, 38, 6489).

A' may be, for example. protected hydroxymethylene.

The process variant (ii) is a standard addition reaction using methods well known to those skilled in the art. The process is preferably carried out in a polar organic solvent e.g. acetonitrile in the presence of an organic base e.g. triethylamine.

In process variant (iii) the coupling may be effected in acetonitrile at room temperature in the presence of one equivalent of lithium perchlorate as catalyst (general method of J.E. Chateauneuf *et al, J. Org. Chem.,* 56, 5939-5942, 1991) or more preferably with ytterbium triflate in dichloromethane. In some cases an elevated temperature such as 40 - 70 °C may be beneficial. Alternatively, the piperazine may be treated with a base, such as one equivalent of butyl lithium, and the resulting salt reacted with the oxirane in an inert solvent such as tetrahydrofuran, preferably at an elevated temperature such as 80°C. Use of a chiral epoxide will afford single diastereomers. Alternatively, mixtures of diastereomers may be separated by preparative HPLC or by conventional resolution through crystallisation of salts formed from chiral acids.

The process variant (iv) is a standard urea formation reaction from the reaction of an isocyanate with an amine and is conducted by methods well known to those skilled in the art (for example see March, J; *Advanced Organic Chemistry, Edition* 3 (John Wiley and Sons, 1985), p802-3). The process is preferably carried out in a polar solvent such as N,N-dimethylformamide.

In process variant (v) the process is two step: firstly a condensation using a base, preferably sodium hydride or alkoxide, sodamide, alkyl lithium or lithium dialkylamide, preferably in an aprotic solvent e.g. ether, THF or benzene; secondly, hydrolysis using an inorganic acid, preferably HCl in aqueous organic solvent at 0-100°C. Analogous routes are described in DE330945, EP31753, EP53964 and H. Sargent, J. Am. Chem. Soc. **68**, 2688-2692 (1946). Similar Claisen methodology is described in Soszko et. al., Pr.Kom.Mat. Przyr.Poznan.Tow.Przyj.Nauk., (1962), 10, 15.

In process variant (vi) the reaction is carried out in the presence of a base, preferably organometallic or metal hydride e.g. NaH, lithium diisopropylamide or NaOEt, preferably in an aprotic solvent, preferably THF, ether or benzene at -78 to 25°C (analogous process in Gutswiller et al. (1978) J. Am. Chem. Soc. 100,576).

In process variants (vii) and (viii) if a base is used it is preferably NaH, KH, an alkyl lithium e.g. BuLi, a metal alkoxide e.g. NaOEt, sodamide or lithium dialkylamide e.g.di- isopropylamide. An analogous method is described in US 3989691 and M.Gates et. al. (1970) J. Amer.Chem.Soc., 92, 205, as well as Taylor et al. (1972) JACS 94, 6218.

In process variant (x) where Y is CHO the reaction is a standard reductive alkylation using, e.g., sodium borohydride or sodium triacetoxyborohydride (Gribble, G. W. in *Encyclopedia of Reagents for Organic Synthesis (Ed. Paquette, L. A.)* (John Wiley and Sons, 1995), p 4649).

The process variant (xi) is a standard alkylation reaction well known to those skilled in the art, for example where an alcohol or amine is treated with an alkyl halide in the presence of a base (for example see March, J; *Advanced Organic Chemistry, Edition* 3 (John Wiley and Sons, 1985), p364-366 and p342-343). The process is preferably carried out in a polar solvent such as N,N-dimethylformamide

In process variant (xii) the reaction is an alkylation, examples of which are described in J. Med. chem. (1979) 22(10) 1171-6. The compound of formula (IV) may be prepared from the corresponding compound where X is NHR^{11'} by acylation with an appropriate derivative of the acid WCH₂COOH such as the acid chloride or sulphonation with an appropriate derivative of the sulphonic acid WCH₂SO₃H such as the sulphonyl chloride.

In process variant (xiii) the reaction is a standard sulphonamide formation reaction well known to those skilled in the art. This may be e.g. the reaction of a sulphonyl halide with an amine.

In process variant (xiv) where X is W such as halogen, methanesulphonyloxy or trifluoromethanesulphonyloxy, the hydroxy group in Y is preferably converted to an OM group where M is an alkali metal by treatment of an alcohol with a base. The base is preferably inorganic such as NaH, lithium diisopropylamide or sodium. Where X is OH, the hydroxy group in Y is activated under Mitsunobu conditions (Fletcher et.al. J Chem Soc. (1995), 623). Alternatively the X=O and Y=CH₂OH groups can be reacted directly by activation with dichlorocarbodiimide (DCC) (Chem. Berichte 1962, 95, 2997 or Angewante Chemie 1963 75, 377).

In process variant (xv) the reaction is conducted in the presence of an organic base such as triethylamine or pyridine such as described by Fuhrman et.al., J. Amer. Chem. Soc.; **67**, 1245, 1945. The X=NR^{11'}SO₂W or Y=SO₂W intermediates can be formed from the requisite amine e.g. by reaction with SO₂Cl₂ analogously to the procedure described by the same authors Fuhrman et.al., J. Amer. Chem. Soc.; **67**, 1245, 1945.

Reduction of a carbonyl group A or B to CHOH can be readily accomplished using reducing agents well known to those skilled in the art, e.g. sodium borohydride in aqueous ethanol or lithium aluminium hydride in ethereal solution. This is analogous to methods described in EP53964, US384556 and J. Gutzwiller *et al, J. Amer. Chem. Soc.,* 1978, 100, 576.

The carbonyl group A or B may be reduced to CH₂ by treatment with a reducing agent such as hydrazine in ethylene glycol, at e.g. 130-160°C, in the presence of potassium hydroxide.

Reaction of a carbonyl group A or B with an organometallic reagent yields a group where R⁶ or R⁸ is OH and R⁷ or R⁹ is alkyl.

A hydroxy group on A or B may be oxidised to a carbonyl group by oxidants well known to those skilled in the art, for example, manganese dioxide, pyridinium chlorochromate or pyridinium dichromate.

A hydroxyalkyl A-B group CHR⁶CR⁸OH or CR⁶(OH)CHR⁸ may be dehydrated to give the group CR⁶=CR⁸ by treatment with an acid anhydride such as acetic anhydride.

Methods for conversion of CR⁶=CR⁸ by reduction to CHR⁶CHR⁸ are well known to those skilled in the art, for example using hydrogenation over palladium on carbon as catalyst. Methods for conversion of CR⁶=CR⁸ to give the A-B group CR⁶(OH)CHR⁸ or CHR⁶CR⁸OH are well known to those skilled in the art for example by epoxidation and subsequent reduction by metal hydrides, hydration, hydroboration or oxymercuration.

An amide carbonyl group may be reduced to the corresponding amine using a reducing agent such as lithium aluminium hydride.

A hydroxy group in A or B may be converted to azido by activation and displacement e.g. under Mitsunobu conditions using hydrazoic acid or by treatment with diphenylphosphorylazide and base, and the azido group in turn may be reduced to amino by hydrogenation.

Examples of groups Z^{1'}, Z^{2'}, Z^{3'}, Z^{4'}, Z^{5'}, are CR^{1a'} where R^{1a'} is a group convertible to R^{1a}. Z^{1'}, Z^{2'}, Z^{3'}, Z⁴' and Z^{5'} are preferably Z¹, Z², Z³, Z⁴ and Z⁵.

R^{1a'} and R^{1'} are preferably R^{1a} and R¹. R^{1'} is preferably methoxy. R^{3'} is R³ or more preferably hydrogen, vinyl, alkoxycarbonyl or carboxy. R^{4'} is R⁴ or more preferably H or an N-protecting group such as t-butoxycarbonyl, benzyloxycarbonyl or 9-fluorenylmethyloxycarbonyl.

Conversions of R^{1a'}, R^{1'}, R^{3'} and R^{4'} and interconversions of R^{1a}, R¹, R³ and R⁴ are conventional. In compounds which contain an optionally substituted hydroxy group, suitable conventional hydroxy protecting groups which may be removed without disrupting the remainder of the molecule include acyl and alkylsilyl groups.

For example R^{1'} methoxy is convertible to R^{1'} hydroxy by treatment with lithium and diphenylphosphine (general method described in Ireland et. al. (1973) J.Amer.Chem.Soc.,7829) or HBr. Alkylation of the hydroxy group with a suitable alkyl derivative bearing a leaving group such as halide and a protected amino, piperidyl, amidino or guanidino group or group convertible thereto, yields, after conversion/deprotection, R¹ alkoxy substituted by optionally N-substituted amino, piperidyl, guanidino or amidino.

R³ alkenyl is convertible to hydroxyalkyl by hydroboration using a suitable reagent such as 9-borabicyclo[3.3.1]nonane, epoxidation and reduction or oxymercuration.

R³ 1,2-dihydroxy can be prepared from R^{3'} alkenyl using osmium tetroxide or other reagents well known to those skilled in the art (see Advanced Organic Chemistry *(Ed. March, J.)* (John Wiley and Sons, 1985), p 732-737 and refs. cited therein) or epoxidation followed by hydrolysis (see Advanced Organic Chemistry *(Ed. March, J.)* (John Wiley and Sons, 1985), p 332,333 and refs. cited therein).

R³ vinyl can be chain extended by standard homologation e.g by conversion to hydroxyethyl followed by oxidation to the aldehyde which is then subjected to a Wittig reaction.

Opening an epoxide R^{3'} group with cyanide anion yields a CH(OH)-CH₂CN group.

Opening an epoxide-containing R^{3'} group with azide anion yields an azide derivative which can be reduced to the amine. Conversion of the amine to a carbamate is followed by ring closure with base to give the 2-oxo-oxazolidinyl containing R³ group.

Substituents on R³ alkyl or alkenyl may be interconverted by conventional methods, for example hydroxy may be derivatised by esterification, acylation or etherification. Hydroxy groups may be converted to halogen, thiol, alkylthio, azido, alkylcarbonyl, amino, aminocarbonyl, oxo, alkylsulphonyl, alkenylsulphonyl or aminosulphonyl by conversion to a leaving group and substitution by the required group, hydrolysis or oxidation as appropriate or reaction with an activated acid, isocyanate or alkoxyisocyanate. Primary and secondary hydroxy groups can be oxidised to an aldehyde or ketone respectively and alkyated with a suitable agent such as an organometallic reagent to give a secondary or tertiary alcohol as appropriate. A carboxylate group may be converted to an hydroxymethyl group by reduction of an ester of this acid with a suitable reducing agent such as lithium aluminium hydride.

Substituted 2-oxo-oxazolidinyl containing R³ groups may be prepared from the corresponding aldehyde by conventional reaction with a glycine anion equivalent, followed by cyclisation of the resulting amino alcohol (M Grauert et al, Ann Chem (1985) 1817, Rozenberg et al, Angew Chem Int Ed Engl (1994) 33(1) 91). The resulting 2-oxo-oxazolidinyl group contains a carboxy group which can be converted to other R¹⁰ groups by standard procedures.

Carboxy groups within R³ may be prepared by Jones' oxidation of the corresponding alcohols CH₂OH using chromium acid and sulphuric acid in water/methanol (E.R.H. Jones et al, J.C.S. 1946,39). Other oxidising agents may be used for this transformation such as sodium periodate catalysed by ruthenium trichloride (G.F.Tutwiler *et al,* J.Med.Chem., 1987, *30*(6), 1094), chromium trioxide-pyridine (G. Just *et al,* Synth. Commun. 1979, *9*(7), 613), potassium permanganate (D.E.Reedich *et al,* J. Org. Chem.,1985,*50*(19),3535, and pyridinium chlorochromate (D. Askin *et al,* Tetrahedron Letters, 1988, 29(3), 277.

The carboxy group may alternatively be formed in a two stage process, with an initial oxidation of the alcohol to the corresponding aldehyde using for instance dimethyl sulphoxide activated with oxalyl chloride (N.Cohen *et al,* J. Am. Chem. Soc., 1983, 105, 3661) or dicyclohexylcarbodiimide (R.M.Wengler, Angew. Chim. Int. Ed. Eng., 1985, 24(2), 77), or oxidation with tetrapropylammonium perruthenate (Ley *et al,* J. Chem.Soc. Chem Commun., 1987, 1625). The aldehyde may then be separately oxidised to the corresponding acid using oxidising agents such as silver (II) oxide (R.Grigg *et al,* J. Chem. Soc. Perkin1, 1983, 1929), potassium permanganate (A.Zurcher, Helv. Chim. Acta., 1987, 70 (7), 1937), sodium periodate catalysed by ruthenium trichloride (T.Sakata *et al,* Bull. Chem. Soc. Jpn., 1988, 61(6), 2025), pyridinium chlorochromate (R.S.Reddy *et al,* Synth. Commun., 1988, 18(51), 545) or chromium trioxide *(R.M.Coates et al,* J. Am. Chem. Soc.,1982, 104, 2198).

An R³ CO₂H group may also be prepared from oxidative cleavage of the corresponding diol, CH(OH)CH₂OH, using sodium periodate catalysed by ruthenium trichloride with an acetontrile-carbontetrachloride-water solvent system (V.S.Martin et *al,* Tetrahedron Letters, 1988, *29*(22), 2701).

R³ groups containing a cyano or carboxy group may also be prepared by conversion of an alcohol to a suitable leaving group such as the corresponding tosylate by reaction with para-toluenesulphonyl chloride (M.R.Bell, J. Med. Chem., 1970, 13, 389), or the iodide using triphenylphosphine, iodine, and imidazole (G. Lange, Synth. Commun., 1990, 20, 1473). The second stage is the displacement of the leaving group with cyanide anion (LA.Paquette et al, J. Org. Chem., 1979, 44 (25), 4603; P.A.Grieco et al, J. Org. Chem., 1988, 53 (16), 3658). Finally acidic hydrolysis of the nitrile group gives the desired acids (H.Rosemeyer et al, Heterocycles, 1985, 23 (10), 2669). The hydrolysis may also be carried out with base e.g. potassium hydroxide (H.Rapoport, J. Org. Chem.,1958, 23, 248) or enzymatically (T. Beard et al, Tetrahedron Asymmetry, 1993, 4(6), 1085).

Other functional groups in R³ may be obtained by conventional conversions of carboxy or cyano groups.

Tetrazoles are conveniently prepared by reaction of sodium azide with the cyano group (e.g. F. Thomas et al, Bioorg. Med. Chem. Lett., 1996, *6* (6), 631; K.Kubo et al, J. Med. Chem., 1993, 36,2182) or by reaction of azidotri-n-butyl stannane with the cyano group followed by acidic hydrolysis (P.L.Ornstein, J. Org. Chem., 1994, *59,* 7682 and J. Med. Chem, 1996, *39* (11), 2219).

The 3-hydroxy-3-cyclobutene-1,2-dion-4-yl group (e.g. R.M.Soll, Bioorg. Med. Chem. Lett., 1993, 3 (4), 757 and W. A. Kinney, J. Med. Chem., 1992, 35 (25), 4720) can be prepared by the following sequence:- (1) a compound where R3 is (CH₂)ₙCHO (n = 0,1,2) is treated with triethylamine, carbon tetrabromide triphenylphosphine to give initially (CH₂)ₙCH=CBr₂; (2) dehydrobromination of this intermediate to give the corresponding bromoethyne derivative (CH₂)ₙC≡CBr (for this 2 stage sequence see D. Grandjean et al, Tetrahedron Letters, 1994, *35* (21), 3529); (3) palladium-catalysed coupling of the bromoethyne with 4-(1-methylethoxy)-3-(tri-n-butylstannyl)cyclobut-3-ene-1,2-dione (Liebeskind et al, J. Org. Chem., 1990, *55,* 5359); (4) reduction of the ethyne moity to -CH2CH2- under standard conditions of hydrogen and palladium on charcol catalysis(see Howard et al, Tetrahedron, 1980, *36,* 171); and finally (4) acidic hydrolysis of the methylethoxyester to generate the corresponding 3-hydroxy-3-cyclobutene-1,2-dione group R.M.Soll, Bioorg. Med. Chem. Lett., 1993, *3* (4), 757).

The tetrazol-5-ylaminocarbonyl group may be prepared from the corresponding carboxylic acid and 2-aminotetrazole by dehydration with standard peptide coupling agents such as 1,1'-carbonyldiimidazole (P. L. Ornstein et al, J. Med Chem, 1996, *39* (11),2232).

The alkyl- and alkenyl-sulphonylcarboxamides are similarly prepared from the corresponding carboxylic acid and the alkyl- or alkenyl-sulphonamide by dehydration with standard peptide coupling agents such as 1,1'-carbonyldiimidazole (P. L. Ornstein et al, J.Med.Chem., 1996, 39 (11), 2232).

The hydroxamic acid groups are prepared from the corresponding acids by standard amide coupling reactions eg N. R. Patel et al, Tetrahedron, 1987, *43* (22), 5375

2,4-thiazolidinedione groups may prepared from the aldehydes by condensation with 2,4-thiazolidinedione and subsequent removal of the olefinic double bond by hydrogenation.

The preparation of 5-oxo-1,2,4-oxadiazoles from nitriles is decribed by Y.Kohara et al, Bioorg. Med. Chem. Lett., 1995, 5(17), 1903.

1,2,4-triazol-5-yl groups may be prepared from the corresponding nitrile by reaction with an alcohol under acid conditions followed by reaction with hydrazine and then an R¹⁰-substituted activated carboxylic acid (see JB Polya in 'Comprehensive Heterocyclic Chemistry' Edition 1 p762, Ed AR Katritzky and CW Rees, Pergamon Press, Oxford 1984 and J.J. Ares et al, J. Heterocyclic Chem., 1991, 28(5), 1197).

NH is converted to NR⁴ by conventional means such as amide or sulphonamide formation for compounds where V is CO or SO₂ by methods well known to those skilled in the art or, where V is CH₂, by alkylation with an alkyl halide in the presence of base, reaction with a vinyl derivatives X⁴-X³-CH=CH₂, for example by heating in an alcohol such as ethanol containing an acid such as acetic acid, acylation/reduction or reductive alkylation with an aldehyde.

Examples of R^{4'} convertible to R⁴ include N-protecting groups and -V-CH₂₋CO₂H which may be reacted with an amine X⁴-NH₂ in the presence of a coupling agent such as 1-(3-dimethylaminopropyl)-3-ethylcarbodiimide (EDC) to give R⁴=-V-CH₂-CO-NH-X⁴. This reaction may be carried out on the compound of formula (V) or after coupling (IV) and (V).

Conventional procedures are used to substitute the piperazine NH with the required group R⁴. For example an oxiranylmethyl group may be introduced by reaction with epichlorohydrin, and the resulting oxirane opened with a suitable amine X⁴-NH₂ to give an R⁴ group of the form -CH₂-CH(OH)-CH₂-NH-X⁴. Cyclisation of the hydroxy and amino (R¹⁴ and R¹³) groups may be effected with phosgene in base. R⁴ groups of the form -CH₂-CH₂-NH-X³-X⁴ may be introduced by alkylation of the piperazine NH. A flexible synthesis involves the introduction of an intermediate group -CH₂-CH₂-NH₂ by treatment with a 2-haloethylcarbamic acid ester followed by reduction. The intermediate group can then be acylated or alkylated as required to introduce the -X³-X⁴ group. R⁴ groups where X³ is O may be prepared from the X⁴-oxirane reacted with an R^{4'} CH₂CH₂OH group in the presence of lithium perchlorate.

Interconversion of substituents on the aromatic group X⁴ may be carried out conventionally at any appropriate point in the synthesis. For example pyridine may be oxidised to the the N-oxide with and oxidising agent such as meta-chloroperbenzoic acid, hydrogen peroxide or t-butyl hydroperoxide after protection of any other nitrogen atoms. N-oxides may be rearranged in trifluoroacetic acid to give the hydroxypyridines.

Where one of R³ and R⁶, R⁷, R⁸ or R⁹ contains a carboxy group and the other contains a hydroxy or amino group they may together form a cyclic ester or amide linkage. This linkage may form spontaneously during coupling of the compound of formula (IV) and the piperazine moiety or in the presence of standard peptide coupling agents.

It will be appreciated that under certain circumstances interconvertions may interfere, for example, hydroxy groups in A or B and the piperazine NH will require protection e.g. as a carboxy- or silyl-ester group for hydroxy and as an acyl derivative for piperidine nitrogen, during conversion of R^{1a'}, R^{1'}, R^{3'} or R^{4'}, or during the coupling of the compounds of formulae (IV) and (V).

Compounds of formulae (IV) and (V) are known compounds, (see for example Smith *et al, J. Amer. Chem. Soc.,* 1946, 68, 1301) or prepared analogously, see for example the references cited above for reaction variant (a).

Compounds of formula (IV) where X is CR⁶R⁷SO₂W may be prepared by a route analogous to that of Ahmed El Hadri *et al, J. Heterocyclic Chem.,* 1993, 30(3), 631. Thus compounds of formula (IV) where X is CH₂SO₂OH may be prepared by reacting the corresponding 4-methyl compound with N-bromosuccinimide, followed by treatment with sodium sulfite. The leaving group W may be converted to another leaving group W, e.g. a halogen group, by conventional methods.

The isocyanate of formula (IV) may be prepared conventionally from a 4-amino derivative such as 4-amino-quinoline, and phosgene, or phosgene equivalent (eg triphosgene) or it may be prepared more conveniently from a 4-carboxylic acid by a "one-pot" Curtius Reaction with diphenyl phosphoryl azide (DPPA) [see T. Shiori et al. *Chem. Pharm. Bull.* **35**, 2698-2704 (1987)].

The 4-amino derivatives are commercially available or may be prepared by conventional procedures from a corresponding 4-chloro derivative by treatment with ammonia (O.G. Backeberg et. al., J. Chem Soc., 381, 1942) or propylamine hydrochloride (R. Radinov et. al., Synthesis, 886, 1986).

4-Alkenyl compounds of formula (IV) may be prepared by conventional procedures from a corresponding 4-halogeno-derivative by e.g. a Heck synthesis as described in e.g. *Organic Reactions,* 1982, 27, 345.

4-Halogeno derivatives of compounds of formula (IV) are commercially available, or may be prepared by methods known to those skilled in the art. A 4-chloroquinoline is prepared from the corresponding quinolin-4-one by reaction with phosphorus oxychloride (POCl₃) or phosphorus pentachloride, PCl₅. A 4-chloroquinazoline is prepared from the corresponding quinazolin-4-one by reaction with phosphorus oxychloride (POCl₃) or phosphorus pentachloride PCl₅. A quinazolinone and quinazolines may be prepared by standard routes as described by T.A. Williamson in *Heterocyclic Compounds,* 6, 324 (1957) Ed. RC. Elderfield.

4-Carboxy derivatives of compounds of formula (IV) are commercially available or may be prepared by conventional procedures for preparation of carboxy heteroaromatics well known to those skilled in the art. For example, quinazolines may be prepared by standard routes as described by T.A. Williamson in *Heterocyclic Compounds,* 6, 324 (1957) Ed. R.C. Elderfield. These 4-carboxy derivatives may be activated by conventional means, e.g. by conversion to an acyl halide or anhydride.

Pyridazines may be prepared by routes analogous to those described in Comprehensive Heterocyclic Chemistry, Volume 3, Ed A.J. Boulton and A. McKillop and napthyridines may be prepared by routes analogous to those described in Comprehensive Heterocyclic Chemistry, Volume 2, Ed A.J. Boulton and A. McKillop.

A 4-oxirane derivative of compounds of formula (IV) is conveniently prepared from the 4-carboxylic acid by first conversion to the acid chloride with oxalyl chloride and then reaction with trimethylsilyldiazomethane to give the diazoketone derivative. Subsequent reaction with 5M hydrochloric acid gives the chloromethylketone. Reduction with sodium borohydride in aqueous methanol gives the chlorohydrin which undergoes ring closure to afford the epoxide on treatment with base, e.g. potassium hydroxide in ethanol-tetrahydrofuran.

Alternatively and preferably, 4-oxirane derivatives can be prepared from bromomethyl ketones which can be obtained from 4-hydroxy compounds by other routes well known to those skilled in he art. For example, hydroxy compounds can be converted to the corresponding 4-trifluoromethanesulphonates by reaction with trifluoromethanesulphonic anhydride under standard conditions (see K. Ritter, Synthesis, 1993, 735). Conversion into the corresponding butyloxyvinyl ethers can be achieved by a Heck reaction with butyl vinyl ether under palladium catalysis according to the procedure of W. Cabri *et al,* J. Org. Chem, 1992, 57 (5), 1481. (Alternatively, the same intermediates can be attained by Stille coupling of the trifluoromethanesulphonates or the analaogous chloro derivatives with (1-ethoxyvinyl)tributyl tin, T. R. Kelly, J. Org. Chem., 1996, **61**, 4623.) The alkyloxyvinyl ethers are then converted into the corresponding bromomethylketones by treatment with N-bromosuccinimide in aqueous tetrahydrofuran in a similar manner to the procedures of J. F. W. Keana, J. Org. Chem., 1983, **48,** 3621 and T. R. Kelly, J. Org. Chem., 1996, **61,** 4623.

The 4-hydroxyderivatives can be prepared from an aminoaromatic by reaction with methylpropiolate and subsequent cyclisation, analogous to the method described in N. E. Heindel et al, J. Het. Chem., 1969, **6**, 77. For example, 5-amino-2-methoxy pyridine can be converted to 4-hydroxy-6-methoxy-[1,5]naphthyridine using this method.

If a chiral reducing agent such as (+) or (-)-B-chlorodiisopinocamphenylborane ['DIP-chloride'] is substituted for sodium borohydride, the prochiral chloromethylketone is converted into the chiral chlorohydrin with ee values generally 85-95% [see C. Bolm *et al, Chem. Ber.* 125, 1169-1190, (1992)]. Recrystallisation of the chiral epoxide gives material in the mother liquor with enhanced optical purity (typically ee 95%).

The *(R)*-epoxide, when reacted with a piperazine derivative gives ethanolamine compounds as single diastereomers with (*R*)-stereochemistry at the benzylic position.

Alternatively, the epoxide may be prepared from the 4-carboxaldehyde by a Wittig approach using trimethylsulfonium iodide [see G.A. Epling and K-Y Lin, *J. Het. Chem.,* 1987, 24, 853-857], or by epoxidation of a 4-vinyl derivative. 4-Hydroxy-1,5-naphthyridines can be prepared from 3-aminopyridine derivatives by reaction with diethyl ethoxymethylene malonate to produce the 4-hydroxy-3-carboxylic acid ester derivative with subsequent hydrolysis to the acid, followed by thermal decarboxylation in quinoline (as for example described for 4-Hydroxy-[1,5]naphthyridine-3-carboxylic acid, J. T. *Adams et al., J.Amer.Chem.Soc.,* 1946, **68**, 1317). A 4-hydroxy-[1,5]naphthyridine can be converted to the 4-chloro derivative by heating in phosphorus oxychloride, or to the 4-methanesulphonyloxy or 4-trifluoromethanesulphonyloxy derivative by reaction with methanesulphonyl chloride or trifluoromethanesulphonic anhydride, respectively, in the presence of an organic base. A 4-amino 1,5-naphthyridine can be obtained from the 4-chloro, 4-methanesulphonyloxy or 4-trifluoromethanesulphonyloxy derivative by reaction with n-propylamine in pyridine.

Similarly, 6-methoxy-1,5-naphthyridine derivatives can be prepared from 3-amino-6-methoxypyridine.

1,5-Naphthyridines may be prepared by other methods well known to those skilled in the art (for examples see P.A. Lowe in "Comprehensive Heterocyclic Chemistry" Volume 2, p581-627, Ed A.R. Katritzky and C.W. Rees, Pergamon Press, Oxford, 1984).

The 4-hydroxy and 4-amino-cinnolines may be prepared following methods well known to those skilled in the art [see A.R. Osborn and K. Schofield, *J. Chem. Soc.* 2100 (1955)]. For example, a 2-aminoacetopheneone is diazotised with sodium nitrite and acid to produce the 4-hydroxycinnoline with conversion to chloro and amino derivatives as described for 1,5-naphthyridines.

The substituted piperazines of formula (V) are either commercially available or may be synthesised by hydrogenation of the corresponding pyrazines (eg von E. Felder et al. *Helv. Chim. Acta* **33,** 888-896 (1960)], or by diborane reduction of a suitable lactam [eg H.L. Larkins et al. *Tet. Lett.* **27,** 2721-2724 (1986)].
Chiral piperazines may be prepared from chiral 2-(S)- and 2-(R)-piperazinecarboxylic acid. Racemic piperazine-2-carboxylic acid (commercially available) may be resolved by crystallisation of the (S)- and (R)-dicainphor-10-sulfonic acid salts [following the general method ofK. Stingl et al. *Tet. Asymmetry* **8**, 979-982 (1997)-described for preparation of 2-(S)-piperazinecarboxylic acid].

Piperazine-2-carboxylic acid may be differentially protected [following the procedure of C.F. Bigge et al. *Tet. Lett.* **30**, 5193-5196 (1989)] by first reacting with 2-(t-butoxycarbonyloxyimino)-2-phenylacetonitrile which selectively reacts on N-4, and then by reacting with benzylchloroformate which reacts on N-1. The 2-carboxylic acid is then methylated (conveniently with TMS-diazomethane). Hydrogenation (over Pd/C) then removes the carbobenzyloxy group, which may be alkylated or acylated with the required R⁴ group as described above for the conversion of R^{4'}=H to R⁴. Reaction with trifluoroacetic acid (optionally in dichloromethane) removes the N-4-butoxycarbonyloxy group to afford the required 4-H piperazine.

The chiral piperazine-2-carboxylic acids may be elaborated to various derivatives, for example, an Arndt-Eistert procedure (involving silver salt mediated rearrangement of a diazoketone) will give chiral 2-acetic acid derivatives (initially via the methyl ester). Reduction of the intermediate ester with standard reducing agents such as lithium aluminium hydride will produce a hydroxymethyl derivative.

Compounds of formula (V) where n=1 may be prepared from the compound where n=0 by homologation eg starting from a compound of formula (V) where Y=CO₂H.

R⁴-halides, vinyl derivatives X⁴-X³-CH=CH₂, acyl derivative X⁴-X³-X²-X¹⁻COW and X⁴-X³-X²-X¹-SO₂W or aldehydes X⁴-X³-X²-X¹-CHO are commercially available or are prepared conventionally. The aldehydes may be prepared by partial reduction of the corresponding ester (see *Reductions by the Alumino- and Borohydrides in Organic Synthesis,* 2nd ed., Wiley, N.Y., 1997; *JOC,* 3197, 1984; *Org. Synth. Coll.,* 102, 1990; 136, 1998; *JOC,* 4260, 1990; *TL,* 995, 1988; *JOC,* 1721, 1999; *Liebigs Ann.*/*Recl.,* 2385, 1997; *JOC,* 5486, 1987) with lithium aluminium hydride or diisobutylaluminium hydride or more preferably by reduction to the alcohol, with lithium aluminium hydride or sodium borohydride, followed by oxidation to the aldehyde with manganese (II) dioxide. The aldehydes may also be prepared from carboxylic acids by conversion to a mixed anhydride for example by reaction with isobutyl chloroformate followed by reduction with sodium borohydride (R. J. Alabaster et al., Synthesis, 598, 1989) to give the benzylic alcohols and then oxidation with a standard oxidising agent such as pyridinium dichromate or manganese (II) dioxide. Acyl derivative may be prepared by activation of the corresponding ester. R⁴-halides such as bromides may be prepared from the alcohol R⁴OH by reaction with phosphorus tribromide in DCM/triethylamine. Where X² is CO and X³ is NR¹³ the R⁴-halide may be prepared by coupling an X⁴-NH₂ amine and bromopropionyl bromide. Vinyl derivatives X⁴-X³⁻CH=CH₂ may be prepared from the corresponding diethylaminoethyl derivative by quaternisation with dimethyl sulphate and heating, resulting in elimination of the charged amino group. The diethylaminoethyl derivative may be prepared from another ethyl derivative eg the hydroxyethyl by conventional amine formation. Alternatively, it may be prepared from a methyl derivative by condensation with diethylamine and formaldehyde.

Conversions of R^{1a'}, R^{1'}, R^{3'} and R^{4'} may be carried out on the intermediates of formulae (IV), (V) and (Vb) prior to their reaction to produce compounds of formula (I) in the same way as described above for conversions after their reaction.

The pharmaceutical compositions of the invention include those in a form adapted for oral, topical or parenteral use and may be used for the treatment of bacterial infection in mammals including humans.

The antibiotic compounds according to the invention may be formulated for administration in any convenient way for use in human or veterinary medicine, by analogy with other antibiotics.

The composition may be formulated for administration by any route, such as oral, topical or parenteral. The compositions may be in the form of tablets, capsules, powders, granules, lozenges, creams or liquid preparations, such as oral or sterile parenteral solutions or suspensions.

The topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams.

The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

Tablets and capsules for oral administration may be in unit dose presentation form, and may contain conventional excipients such as binding agents, for example syrup, acacia, gelatin, sorbitol, tragacanth, or polyvinylpyrollidone; fillers, for example lactose, sugar, maize-starch, calcium phosphate, sorbitol or glycine; tabletting lubricants, for example magnesium stearate, talc, polyethylene glycol or silica; disintegrants, for example potato starch; or acceptable wetting agents such as sodium lauryl sulphate. The tablets may be coated according to methods well known in normal pharmaceutical practice. Oral liquid preparations may be in the form of, for example, aqueous or oily suspensions, solutions, emulsions, syrups or elixirs, or may be presented as a dry product for reconstitution with water or other suitable vehicle before use. Such liquid preparations may contain conventional additives, such as suspending agents, for example sorbitol, methyl cellulose, glucose syrup, gelatin, hydroxyethyl cellulose, carboxymethyl cellulose, aluminium stearate gel or hydrogenated edible fats, emulsifying agents, for example lecithin, sorbitan monooleate, or acacia; non-aqueous vehicles (which may include edible oils), for example almond oil, oily esters such as glycerine, propylene glycol, or ethyl alcohol; preservatives, for example methyl or propyl p-hydroxybenzoate or sorbic acid, and, if desired, conventional flavouring or colouring agents.

Suppositories will contain conventional suppository bases, e.g. cocoa-butter or other glyceride.

For parenteral administration, fluid unit dosage forms are prepared utilizing the compound and a sterile vehicle, water being preferred. The compound, depending on the vehicle and concentration used, can be either suspended or dissolved in the vehicle. In preparing solutions the compound can be dissolved in water for injection and filter sterilised before filling into a suitable vial or ampoule and sealing.

Advantageously, agents such as a local anaesthetic, preservative and buffering agents can be dissolved in the vehicle. To enhance the stability, the composition can be frozen after filling into the vial and the water removed under vacuum. The dry lyophilized powder is then sealed in the vial and an accompanying vial of water for injection may be supplied to reconstitute the liquid prior to use. Parenteral suspensions are prepared in substantially the same manner except that the compound is suspended in the vehicle instead of being dissolved and sterilization cannot be accomplished by filtration. The compound can be sterilised by exposure to ethylene oxide before suspending in the sterile vehicle. Advantageously, a surfactant or wetting agent is included in the composition to facilitate uniform distribution of the compound.

The compositions may contain from 0.1% by weight, preferably from 10-60% by weight, of the active material, depending on the method of administration. Where the compositions comprise dosage units, each unit will preferably contain from 50-500 mg of the active ingredient. The dosage as employed for adult human treatment will preferably range from 100 to 3000 mg per day, for instance 1500 mg per day depending on the route and frequency of administration. Such a dosage corresponds to 1.5 to 50 mg/kg per day. Suitably the dosage is from 5 to 20 mg/kg per day.

No toxicological effects are indicated when a compound of formula (I) or a pharmaceutically acceptable salt or *in vivo* hydrolysable ester thereof is administered in the above-mentioned dosage range.

The compound of formula (I) may be the sole therapeutic agent in the compositions of the invention or a combination with other antibiotics or with a β-lactamase inhibitor may be employed.

Compounds of formula (I) are active against a wide range of organisms including both Gram-negative and Gram-positive organisms.

The following examples illustrate the preparation of certain compounds of formula (I) and the activity of certain compounds of formula (I) against various bacterial organisms.

### Examples

### Example 1 (R)-1-(3,5-Difluoro-phenylamino)-3-{4-[(R)-2-hydroxy-2-(6-methoxy-quinolin-4-yl)-ethyl]-piperazin-1-yl}-propan-2-ol dioxalate salt

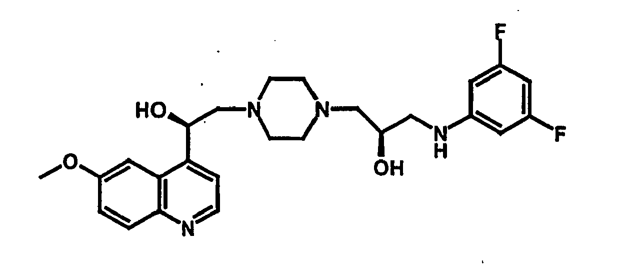

### (a) 6-Methoxyquinoline-4-carboxylic acid

The title compound was prepared by modification of the procedure described by W.E. Daering and J.D. Chanley, *J. Amer. Chem. Soc.,* 1946, **68**, 586. A mixture of Quinine (225g, 0.70 mol), *tert*-butanol (1 litre) and water (10 ml) was treated with potassium *tert*-butoxide (170g, 1.5 mol). The mixture was stirred at 30°C, while air was bubbled through for 3 days. The mixture was diluted with diethyl ether and water and the layers separated. The aqueous phase was extracted with ethyl acetate. The combined diethyl ether and ethyl acetate extracts were dried over magnesium sulfate and evaporated to give recovered starting material (approximately 100g). The aqueous phase was acidified to pH5 with 5M hydrochloric acid. The precipitate was collected by filtration, washed with water and methanol, then dried to give 6-methoxyquinoline-4-carboxylic acid as a yellow solid (64.6g, 46%).
δH (d-6 DMSO), 6.23-5.95 (1H, m), 5.34-5.06 (2H, m), 3.37-2.92 (5H, m), 2.70 (1H, m), 2.38-2.15 (3H, m), 1.94-1.52 (2H, m).

### (b) (R)-2-(6-Methoxyquinolin-4-yl)oxirane

A solution of 6-methoxyquinoline-4-carboxylic acid (10g) in dichloromethane was heated under reflux with oxalyl chloride (5ml) and N,N'-dimethylformamide (2 drops) for 1 hour and evaporated to dryness. The residue, in dichloromethane (100ml) was treated with a 2M solution of trimethylsilyldiazomethane in hexane (50ml) and stirred at room temperature for 18 hours. 5M Hydrochloric acid (150ml) was added and the solution was stirred at room temperature for 3 hours. It was basified with sodium carbonate solution, extracted with ethyl acetate and chromatographed on silica gel eluting with ethyl acetate-hexane to give the chloromethyl ketone (4.2g). A batch of the chloromethyl ketone (20g) was reduced with (+)-B-chlorodiisopinocamphenylborane (40g) in dichloromethane (400ml) at room temperature for 18 hours followed by treatment with diethanolamine (30g) for 3 hours. The product was chromatographed on silica gel eluting with ethyl acetate-hexane to give the chloroalcohol (16.8g), which was dissolved in tetrahydrofuran (100 ml) and reacted with sodium hydroxide (2.6g) in water (13ml) for 1.5 hours. The reaction mixture was evaporated to dryness and chromatographed on silica gel eluting with ethyl acetate/hexane to give the title compound as a solid (10.4 g) (84% ee by chiral HPLC).
Recrystallisation from ether-pentane gave mother-liquor (7.0 g) (90% ee).
MS (+ve ion electrospray) m/z 202 (MH+)
The absolute stereochemistry was defined to be *(R)* by an NMR study on the Mosher's esters derived from the product obtained by reaction with 1-*tert*-butylpiperazine.

### (c) 4-[(R)-2-Hydroxy-2-(6-methoxy-quinolin-4-yl)-ethyl]-piperazine-1-carboxylic acid tert-butyl ester

*(R)*-2-(6-Methoxyquinolin-4-yl)oxirane (4.30g, 21.37mmol) was dissolved in acetonitrile (30mL). To the solution was added piperazine-1-carboxylic acid *tert*-butyl ester (7.17g, 38.47mmol) and lithium perchlorate (2.27g, 106.4mmol). The resulting slurry was stirred at room temperature for 10 hours and then concentrated *in vacuo.* The residue was partitioned between ethyl acetate and water and the organic phase dried over magnesium sulfate. Concentration *in vacuo* afforded a colourless oil which was subjected to purification by column chromatography on silica gel using a dichloromethane/methanol gradient. This provided the desired compound as a colourless oil (7.65g, 92%).
MS (APCI+) m/z 388 (MH+).

### (d) (R)-1-(6-Methoxy-quinolin-4-yl)-2-piperazin-1-yl-ethanol

4-[*(R)*-2-Hydroxy-2-(6-methoxy-quinolin-4-yl)-ethyl]-piperazine-1-carboxylic acid *tert*-butyl ester (3.50g) was dissolved in dichloromethane (10mL) and trifluoroacetic acid (10mL) was added. The resulting solution was stirred at room temperature for 5 hours and then concentrated *in vacuo.* The residue was purified on silica gel, eluting with methanol and dichloromethane. This provided the desired compound which was used without further purification.
MS (APCI+) m/z 288 (MH+).

### (e) (R)-1-(6-Methoxy-quinolin-4-yl)-2-(4-(S)-1-oxiranylmethyl-piperazin-1-yl)-ethanol

*(R)*-1-(6-Methoxy-quinolin-4-yl)-2-piperazin-1-yl-ethanol (1.80g, 2.50mmol) was dissolved in a 1:1 mixture of tetrahydrofuran and water (5mL). (*R*)-epichlorohydrin (280mg, 3.01mmol) was added and the solution stirred for 5 hours at room temperature. The solution was concentrated *in vacuo* and the residue partitioned between ethyl acetate and saturated aqueous sodium bicarbonate. The organic phase was dried over magnesium sulfate and concentrated *in vacuo.* The residue was subjected to chromatography on silica gel with methanol and dichloromethane to provide a colourless oil (240mg, 11%).
MS (APCI+) m/z 344 (MH+).

### (f) Title compound

*(R)*-1-(6-Methoxy-quinolin-4-yl)-2-(4-*(S)*-1-oxiranylmcthyl-piperazin-1-yl)-ethanol (117mg, 0.341mmol) was dissolved in ethanol (3mL) and then 3,5-difluoroaniline (88mg, 0.682mmol) was added. The resulting solution was heated to 80°C for 10 hours. The solution was concentrated *in vacuo* and the residue subjected to chromatography on silica gel using methanol and dichloromethane as eluant. This afforded the desired compound as a colourless oil (19mg, 12%).
δH (CD₃OD, 250MHz) 8.65-8.23 (1H, d), 7.93-7.89 (1H, m), 7.67-7.87 (1H, d), 7.41-7.37 (2H, m), 6.21-6.14 (2H, m), 6.06-5.97 (1H, m), 5.62-5.46 (1H, dd), 3.93 (3H, s), 3.89-3.85, 3.22-3.00, 2.90-2.45 (15H, m).
MS (APCI+) m/z 473 (MH+).

A solution of the oil (19mg) in dichloromethane (1mL) was added to oxalic acid (7mg) in diethyl ether (10mL) to generate the dioxalate salt. The title compound was isolated by centrifugation, washing with diethyl ether and subsequent drying *in vacuo.*

### Example 2 (R/S)-1-(3,5-Difluoro-phenylamino)-3-{4-[(R)-2-hydroxy-2-(6-methoxy-quinolin4-yl)-ethyl]-piperazin-1-yl}-propan-2-ol

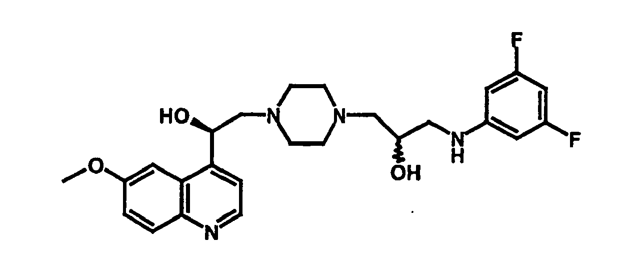

The title compound was prepared in the same manner as *(R)*-1-(3,5-Difluoro-phenylamino)-3-{4-[*(R)*-2-hydroxy-2-(6-methoxy-quinolin-4-yl)-ethyl]-piperazin-1-yl}-propan-2-ol but starting from racemic epichlorohydrin.
MS (APCI+) m/z 473 (MH+).

### Example 3 3-(3,5-Difluoro-phenyl)-5-{4-[(R)-2-hydroxy-2-(6-methoxy-quinolin-4-yl)-ethyl]-piperazin-1-ylmethyl}-oxazolidin-2-one dimesylate salt

(*R*/*S*)-1-(3,5-Difluoro-phenylamino)-3-{4-[*(R)*-2-hydroxy-2-(6-methoxy-quinolin-4-yl)-ethyl]-piperazin-1-yl}-propan-2-ol (Example 2) was dissolved in dichloromethane (1mL) and triethylamine (0.02mL, 0.16mmol) was added followed by phosgene (20% in toluene) (0.04mL, 0.08mmol). The solution was stirred at room temperature for 10 hours and then concentrated *in vacuo.* The residue was partitioned between ethyl acetate and saturated aqueous sodium bicarbonate. The organic phase was dried over magnesium sulfate and the volatiles removed under reduced pressure. The resulting oil was purified on silica gel using methanol and dichloromethane as eluant. This afforded the desired compound as a yellow oil (6mg, 15%).
δH (CD₃OD, 250MHz) 8.79-8.77 (1H, m), 8.07-8.03 (1H, m), 7.65-7.63 (1H, m), 7.37-7.36 (2H, m), 7.18-7.16 (2H, m), 6.63-6.56 (1H, m), 5.48-5.44 (1H, m), 4.81 (1H, m), 3.94 (3H, s), 4.04-3.65, 2.95-2.50 (14H, m).
MS (APCI+) m/z 499 (MH+).

A solution of the oil (6mg) in dichloromethane (1mL) was added to a 0.1M solution of methanesulfonic acid in diethyl ether (0.24mL) in more diethyl ether (10mL) to generate the dimesylate salt. The title compound was isolated by centrifugation, washing with diethyl ether and subsequent drying *in vacuo.*

### Example 4 N-(2-{4-[(R)-2-Hydroxy-2-(6-methoxy-quinolin-4-yl)-ethyl]-piperazin-1-yl}-ethyl)-benzamide dioxalate salt

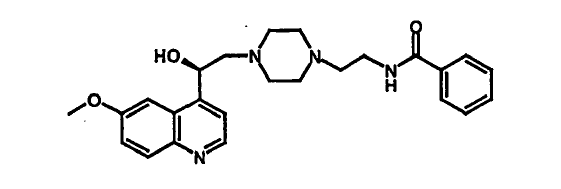

### (a) (2-{4-[(R)-2-Hydroxy-2-(6-methoxy-quinolin-4-yl)-ethyl]-piperazin-1-yl}-ethyl)-carbamic acid benzyl ester

*(R)*-1-(6-Methoxy-quinolin-4-yl)-2-piperazin-1-yl-ethanol (Example 1d) (2.60g, 9.04mmol) was slurried in benzene (20 mL) and *N,N'*-dimethylformamide (6 mL). Potassium carbonate (1.87g, 13.57mmol) was added followed by (2-bromo-ethyl)-carbamic acid benzyl ester (3.50g, 13.57mmol). The resulting slurry was heated to 70°C for 10 hours under argon. Concentration *in vacuo* was followed by extraction into ethyl acetate then drying of the organic phase over magnesium sulfate. Concentration *in vacuo* provided an oil which was subjected to chromatography on silica gel. This afforded the desired product (1.01g, 24%).
MS (APCI+) m/z 465 (MH+).

### (b) (R)-2-[4-(2-Amino-ethyl)-piperazin-1-yl]-1-(6-methoxy-quinolin-4-yl)-ethanol

(2- {4-[*(R)*-2-Hydroxy-2-(6-methoxy-quinolin-4-yl)-ethyl]-piperazin-1-yl}-ethyl)-carbamic acid benzyl ester (1.00g, 2.16mmol) was dissolved in methanol (10mL) and treated with 10% Pd-C catalyst (20mg) then hydrogenated under 1 atmosphere of hydrogen gas at room temperature for 10 hours. The catalyst was removed by filtration through celite^{®} washing with methanol (20mL) and the filtrate concentrated *in vacuo.* This afforded the desired product (583mg, 82%) which was used without further purification.
MS (APCI+) m/z 331 (MH+).

### (c) Title compound

(*R*)-2-[4-(2-Amino-ethyl)-piperazin-1-yl]-1-(6-methoxy-quinolin-4-yl)-ethanol (55mg, 0.166mmol) was dissolved in *N,N'*-dimethylformamide (0.5 mL). 1-(3-Dimethylaminopropyl)-3-ethylcarbodiimide hydrochloride (48mg, 0.249mmol), benzoic acid (30mg, 0.249mmol) and triethylamine (0.07mL, 0.498mmol) were then added and the resulting slurry stirred at room temperature for 10 hours. The solvent was removed in vacuo and the residue subjected to column chromatography on silica gel. This afforded the desired amide (24mg, 33%).
δH (CD₃OD, 250MHz), 8.68-8.66 (1H, d), 7.97-7.41 (9H, m), 5.68-5.63 (1H, dd), 3.96 (3H, s), 3.61-3.56 (2H, m), 2.87-2.71 (12H, m).
MS (APCI+) m/z 436 (MH+).

A solution of the oil (24mg) in dichloromethane (1mL) was added to oxalic acid (10mg) in diethyl ether (10mL) to generate the dioxalate salt. The title compound was isolated by centrifugation, washing with diethyl ether and subsequent drying *in vacuo.*

### Example 5 3,5-Difluoro-N-(2-{4-[(R)-2-hydroxy-2-(6-methoxy-quinolin-4-yl)-ethyl]-piperazin-1-yl}-ethyl)-benzamide dioxalate salt

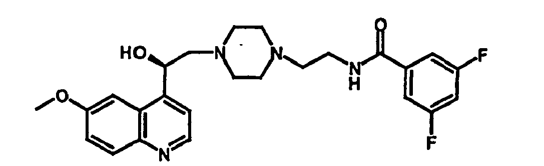

The title compound was prepared in the same manner as Example 4. This afforded the desired compound (33mg, 42%).
MS (APCI+) m/z 471 (MH+).

The corresponding dioxalate salt was prepared in the same manner as Example 1.

### Example 6 Pyridine-2-carboxylic acid (2-{4-[(R)-2-hydroxy-2-(6-methoxy-quinolin-4-yl)-ethyl]-piperazin-1-yl}-ethyl)-amide dioxalate salt

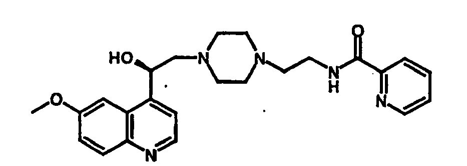

The title compound was prepared in the same manner as Example 4. This afforded the desired compound (11mg, 15%).
MS (APCI+) m/z 436 (MH+).

The corresponding dioxalate salt was prepared in the same manner as Example 1.

### Example 7 Thiophene-2-carboxylic acid (2-{4-[(R)-2-hydroxy-2-(6-methoxy-quinolin-4-yl)-ethyl]-piperazin-1-yl}-ethyl)-amide dioxalate salt

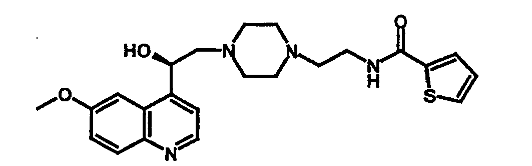

The title compound was prepared in the same manner as Example 4. This afforded the desired compound (13mg, 18%).
MS (APCI+) m/z 441 (MH+).

The corresponding dioxalate salt was prepared in the same manner as Example 1.

### Example 8 Furan-2-carboxylic acid (2-{4-[(R)-2-hydroxy-2-(6-methoxy-quinolin-4-yl)-ethyl]-piperazin-1-yl}-ethyl)-amide dioxalate salt

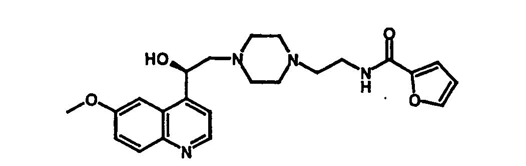

The title compound was prepared in the same manner as Example 4. This afforded the desired compound (24mg, 34%).
MS (APCI+) m/z 425 (MH+).

The corresponding dioxalate salt was prepared in the same manner as Example 1.

### Example 9 3-Cyano-N-(2-{4-[(R)-2-hydroxy-2-(6-methoxy-quinolin-4-yl)-ethyl]-piperazin-1-yl}-ethyl)-benzamide dioxalate salt

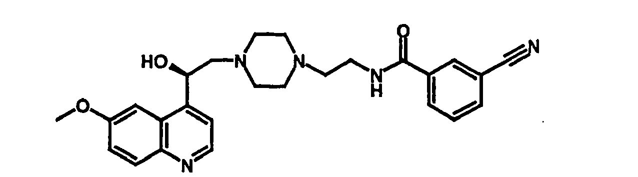

The title compound was prepared in the same manner as Example 4. This afforded the desired compound (25mg, 33%).
MS (APCI+) m/z 460 (MH+).

The corresponding dioxalate salt was prepared in the same manner as Example 1.

### Example 10 N-(2-{4-[(R)-2-Hydroxy-2-(6-methoxy-quinolin-4-yl)-ethyl]-piperazin-1-yl}-ethyl)-trifluoromethyl-benzamide dioxalate salt

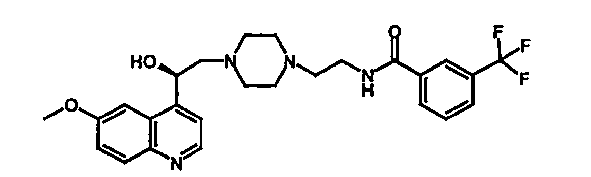

The title compound was prepared in the same manner as Example 4. This afforded the desired compound (25mg, 30%).
MS (APCI+) m/z 503 (MH+).

The corresponding dioxalate salt was prepared in the same manner as Example 1.

### Example 11 (E)-1-{4-[(R)-2-Hydroxy-2-(6-methoxy-quinolin-4-yl)-ethyl]-piperazin-1-yl}-4-phenyl-but-3-en-1-one dioxalate salt

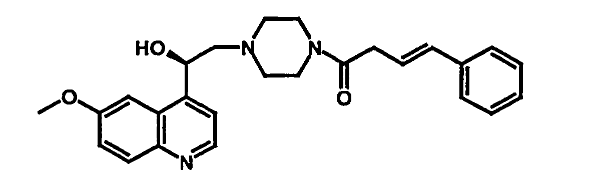

(*R*)-1-(6-Methoxy-quinolin-4-yl)-2-piperazin-1-yl-ethanol (Example 1d) (275mg, 0.96mol) was dissolved in *N,N'*-dimethylformamide (2mL). 1-(3-Dimethylaminopropyl)-3-ethylcarbodiimide hydrochloride (276mg, 1.44mmol), styrylacetic acid (233mg, 1.44mmol) and triethylamine (0.40mL, 2.87mmol) were then added and the resulting slurry stirred at room temperature for 10 hours. The solvent was removed in vacuo and the residue subjected to column chromatography on silica gel. This afforded the desired amide (56mg, 14%). δH (CDCl₃, 250MHz), 8.79-8.77 (1H, d), 8.08-8.04 (1H, d), 7.65-7.63 (1H, d), 7.40-7.13 (7H, m), 6.56-6.26 (2H, m), 5.51-5.46 (1H, dd), 3.90 (3H, s), 3.90-3.30, 2.88-2.48 (12H, m).
MS (APCI+) m/z 432 (MH+).

A solution of the oil (18.5mg) in dichloromethane (1mL) was added to oxalic acid (8mg) in diethyl ether (10mL) to generate the dioxalate salt. The title compound was isolated by centrifugation, washing with diethyl ether and subsequent drying *in vacuo.*

### Example 12 (R)-1-(6-Methoxy-quinolin-4-yl)-2-[4-(4-phenyl-butyl)-piperazin-1-yl]-ethanol dioxalate (a) 4-Phenyl-butan-1-al

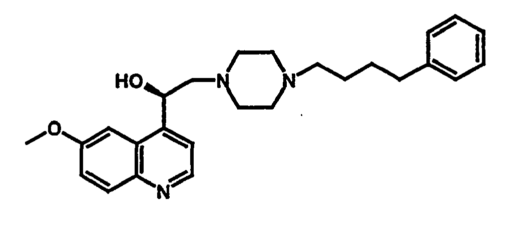

4-Phenyl-butan-1-ol (1.50g, 9.99mmol) was dissolved dichloromethane (10mL) and pyridinium chlorochromate (3.45g, 15.98mmol) was added. The slurry was stirred at room temperature for 1 hour and then filtered through silica gel eluting with an ethyl acetate/hexanes gradient. The desired compound was isolated as a yellow oil. MS (APCI+) m/z 147 (M+-1).

### (b) Title compound

*(R)*-1-(6-Methoxy-quinolin-4-yl)-2-piperazin-1-yl-ethanol (Example 1d) (410mg, 1.42mmol) was dissolved in dichloromethane (2mL) and methanol (0.5mL) and 4-phenyl-butan-1-al (253mg, 1.71mmol) was added. The solution was stirred at room temperature over 4Å molecular seives for 10 hours and then sodium triacetoxyborohydride (454mg, 2.14mmol) was added. The resulting slurry was stirred at room temperature for a further 5 hours. The volatiles were then removed *in vacuo* and the residue subjected to purification by column chromatography on silica gel eluting with a methanol/dichloromethane gradient. This provided the desired compound as a yellow oil (271mg, 45%)
δH (CDCl₃, 250MHz) 8.76-8.75 (1H, d), 8.05-8.01 (1H, d), 7.65-7.63 (1H, d), 7.39-7.16 (7H, m), 5.47-5.41(1H, dd), 3.91 (3H, s), 2.87-2.49 (14H, m), 1.71-1.48 (4H, m).
MS (APCI+) m/z 420 (MH+).

A solution of the oil (41mg) in dichloromethane (1mL) was added to oxalic acid (18.5mg) in diethyl ether (10mL) to generate the dioxalate salt. The title compound was isolated by centrifugation, washing with diethyl ether and subsequent drying *in vacuo.*

### Example 13 (S)-1-(6-Methoxy-quinolin-4-yl)-2-[4-(4-phenyl-butyl)-piperazin-1-yl]-ethylamine trioxalate salt

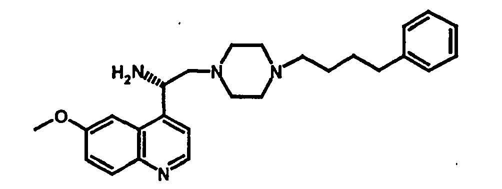

### (a) 4-{(S)-1-Azido-2-[4-(4-phenyl-butyl)-piperazin-1-yl]-ethyl}-6-methoxy-quinoline

*(R)*-1-(6-Methoxy-quinolin-4-yl)-2-piperazin-1-yl-ethanol (Example 1d) (148mg, 0.352mmol) was slurried in a mixture of toluene (3.5mL) and dichloromethane (0.5mL). Diphenyl phosphoryl azide (0.23mL, 1.06mmol) was added followed by 1,8-diazabicyclo[5.4.0]undec-7-ene (0.16mL, 1.06mmol). The slurry was stirred for 10 hours at room temperature after which time it was concentrated under reduced pressure. The residue was subjected to purification on silica gel eluting with a gradient of dichloromethane and methanol. This afforded the desired product as a yellow oil (37mg, 24%).
MS (APCI+) m/z 445 (MH+).

### (b) Title compound

4- {*(S)*-1-Azido-2-[4-(4-phenyl-butyl)-piperazin-1-yl]-ethyl} -6-methoxy-quinoline was dissolved in ethanol (2mL) and 10% palladium on carbon (20mg) was added. The slurry was hydrogenated under one atmosphere of hydrogen at room temperature. After 10 hours the catalyst was removed by filtration through celite^{®} washing with ethanol. The filtrate was concentrated under reduced pressure and the residue purified on silica gel using a dichloromethane/methanol gradient. This provided the desired compound as a colourless oil (10mg, 29%).
δH (CD₃OD, 250MHz), 8.68-8.66 (1H, d), 7.96-7.93 (1H, d), 7.66-7.65 (1H, d), 7.46-7.05 (7H, m), 5.01-4.96 (1H, dd), 3.97 (3H, s), 2.79-2.34 (14H, m), 1.64-1.59 (4H, m). MS (APCI+) m/z 419 (MH+).

A solution of the oil (10mg) in dichloromethane (1mL) was added to oxalic acid (6.5mg) in diethyl ether (10mL) to generate the trioxalate salt. The title compound was isolated by centrifugation, washing with diethyl ether and subsequent drying *in vacuo.*

### Example 14 N-(3,5-Difluoro-phenyl)-3-{4-[(R)-2-hydroxy-2-(6-methoxy-quinolin-4-yl)-ethyl]-piperazin-1-yl}-propionamide dioxalate salt

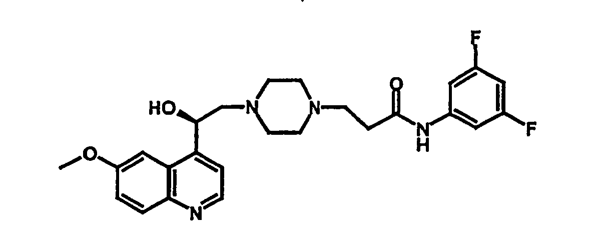

3-{4-[*(R)*-2-Hydroxy-2-(6-methoxy-quinolin-4-yl)-ethyl]-piperazin-1-yl}-propionic acid *tert*-butyl ester (Example lc) (800mg, 1.93mmol) was dissolved in dichloromethane (10mL) and trifluoroacetic acid (10mL) was added. The resulting solution was stirred at room temperature for 2 hours and then concentrated *in vacuo.* The resulting TFA salt (121mg) was dissolved in *N,N*'-dimethylformamide (1mL). To the solution was added 3,5-difluoroaniline (40mg, 0.31mmol) and 1-(3-dimethylaminopropyl)-3-ethylcarbodiimide hydrochloride (60mg, 0.31mmol). This was followed by the addition of triethylamine (0.09mL, 0.62mmol). The solution was stirred at room temperature for 5 hours and then concentrated *in vacuo.* The residue was purified on neutral alumina using a dichloromethane/methanol gradient. This afforded the desired compound as a colourless oil (16mg, 16%).
δH (CD₃OD, 250MHz), 8.49-8.47 (1H, d), 7.77-7.73 (1H, m), 7.51-7.49 (1H, d), 7.25-7.22 (3H, m), 7.10-7.01 (1H, m), 6.50-6.41 (1H, m), 5.49-5.46 (1H, m), 3.77 (3H, s), 3.20-2.40 (14H).
MS (APCI+) m/z 471 (MH+).

A solution of the oil (16mg) in dichloromethane (1mL) was added to oxalic acid (6mg) in diethyl ether (10mL) to generate the dioxalate salt. The title compound was isolated by centrifugation, washing with diethyl ether and subsequent drying *in vacuo.*

### Example 15 1-{4-[(R)-2-Hydroxy-2-(6-methoxy-quinolin-4-yl)-ethyl]-piperazin-1-yl}-3-phenoxy-propan-2-ol dioxalate salt

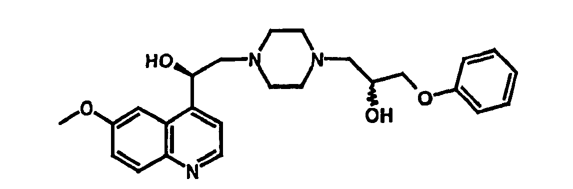

*(R)*-1-(6-Methoxy-quinolin-4-yl)-2-piperazin-1-yl-ethanol (Example 1d) (258mg, 0.899mmol) was dissolved in acetonitrile (4mL). To the resulting solution was added 2-phenoxymethyl oxirane (150mg, 0.989mmol) and lithium perchlorate (105mg, 0.989mmol). The solution was stirred at 50°C for 10 hours and then concentrated under reduced pressure. The residue was subjected to purification by column chromatography on silica gel using a dichloromethane/methanol gradient. This allowed isolation of the desired product (63mg, 16%).
δH (CD₃OD, 250MHz), 8.70-8.69 (1H, d), 7.98-7.93 (2H, m), 7.75-7.73 (1H, m), 7.45-6.94 (6H, m), 5.75-5.72 (1H, m), 4.65-4.60 (1H, m), 3.92 (3H, s), 3.50-2.75 (14H, m). MS (APCI+) m/z 438 (MH+).

A solution of the oil (43mg) in dichloromethane (1mL) was added to oxalic acid (18mg) in diethyl ether (10mL) to generate the dioxalate salt. The title compound was isolated by centrifugation, washing with diethyl ether and subsequent drying *in vacuo.*

### Example 16 3-(3-Fluoro-phenyl)-5-{4-[(R)-2-hydroxy-2-(6-methoxy-quinolin-4-yl)-ethyl]-piperazin-1-ylmethyl}-oxazolidin-2-one dimesylate

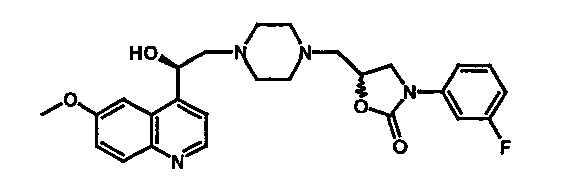

### (a) (R/S)-5-Bromomethyl-3-(3-fluoro-phenyl)-oxazolidin-2-one

Lithium bromide (32 mg, 0.362mmol) and tri-n-butyl phosphine (111mg, 0.507mmol) were heated in xylene (5mL) to 140°C for 1 hour. (*R*/*S*)-2-Bromomethyl-oxirane (0.62mL, 7.25mmol) and 3-fluorophenyl isocyanate (0.993g, 7.25mmol) were added and the mixture was heated to 140°C for 3 hours and then partitioned between ethyl acetate and brine. The organic phase was dried over magnesium sulfate and concentrated *in vacuo.* The residue was subjected to purification by column chromatography on silica gel using an ethyl acetate/hexanes gradient. This afforded the desired compound as a colourless oil (1.75g, 88%).

### (b) (R/S)-4-(2-Oxo-3-phenyl-oxazolidin-5-ylmethyl)-piperazine-1-carboxylic acid tert-butyl ester

(*R*/*S*)-5-Bromomethyl-3-(3-fluoro-phenyl)-oxazolidin-2-one (0.894g, 3.251mmol) was dissolved in N,N'-dimethylformamide (10mL) and piperazine-1-carboxylic acid benzyl ester (0.715g, 3.251mmol) and diisopropyl ethylamine (0.68mL, 3.90mmol) were added. The solution was stirred at 60°C for 4 hours and the the volatiles removed *in vacuo.* The residue was purified by column chromatography on silica gel using as eluant an ethyl acetate/hexanes gradient. This provided the desired product as a colourless oil, (0.938g, 70%).

### (c) (R/S)-3-Phenyl-5-piperazin-1-ylmethyl-oxazolidin-2-one

(*R*/*S*)-4-(2-Oxo-3-phenyl-oxazolidin-5-ylmethyl)-piperazine-1-carboxylic acid *tert*-butyl ester (0.93g, 2.25mmol) was hydrogenated in ethanol (50mL) containing 10% Pd-C catalyst (0.1g). The reaction was conducted at room temperature for 12 hours after which time the catalyst was removed by filtration through a pad of Celite^{®}. The filtrate was concentrated *in vacuo* and the residue used without further purification.

### (d) Title compound

(*R*)-2-(6-Methoxyquinolin-4-yl)oxirane (Example 1b) (140mg, 0.699mmol) was dissolved in acetonitrile (10mL) and (R/S)-3-Phenyl-5-piperazin-1-ylmethyl-oxazolidin-2-one (195mg, 0.699minol) was added followed by lithium perchlorate (74mg, 0.699mmol). The slurry was stirred at room temperature for 12 hours after which time the volatiles were removed and the residue partitioned between brine and ethyl acetate. The organic phase was dried over magnesium sulfate and then concentrated *in vacuo.* The residue was purified on silica gel using a methanol/dichloromethane eluant. This provided a mixture of the title diastereomeric compounds as a colourless oil (47mg, 14%).
δH (CD₃OD, 250MHz) 8.80-8.72 (1H, m), 8.06-8.02 (1H, m), 7.63-7.60 (1H, m), 7.45-7.17 (5H, m), 6.88-6.82 (1H, m), 5.61-5.56 and 5.48-5.46 (1H, m), 4.87-4.70 (1H, m), 3.96 and 3.95 (3H, s), 4.15-3.65 and 2.90-2.45 (14H, m).
MS (APCI+) m/z 481 (MH+).

To a solution of the oil (47mg) in dichloromethane (1mL) was added methane sulfonic acid (1.96mL of a 0.1M solution) in diethyl ether (10mL) to generate the dimesylate salt. The title compound was isolated by centrifugation, washing with diethyl ether and subsequent drying *in vacuo.*

### Example 17 (S)-1-{4-[(R)-2-Hydroxy-2-(6-methoxy-quinolin-4-yl)-ethyl]-piperazin-1-yl}-3-phenylamino-propan-2-ol dioxalate

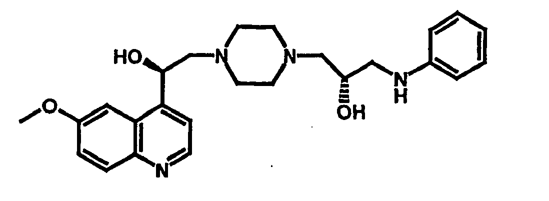

### (a) (R)-1-(6-Methoxy-quinolin-4-yl)-2-(4-(R)-1-oxiranylmethyl-piperazin-1-yl)-ethanol

This compound was prepared in the same manner as (*R*)-1-(6-Methoxy-quinolin-4-yl)-2-(4-(*S*)-1-oxiranylmethyl-piperazin-1-yl)-ethanol (Example le) beginning from (*S*)-epichlorohydrin.

### (b) Title compound

(*R*)-1-(6-Methoxy-quinolin-4-yl)-2-(4-(*R*)-1-oxiranylmethyl-piperazin-1-yl)-ethanol (10mg, 0.03mmol) was dissolved in ethanol (0.5mL) and aniline (20mg, 0.22mmol) was added. The solution was stirred at 60°C for 12 hours after which time the volatiles were removed *in vacuo* and the residue purified by column chromatography on silica gel. Eluting with a gradient of methanol/dichloromethane afforded the desired product (12mg, 94%).
MS (APCI+) m/z 436 (MH+).

A solution of the oil (8mg) in dichloromethane (1mL) was added to oxalic acid (3.5mg) in diethyl ether (10mL) to generate the dioxalate salt. The title compound was isolated by centrifugation, washing with diethyl ether and subsequent drying *in vacuo.*

### Biological Activity

The MIC (µg/ml) of test compounds against various organisms was determined: **S. aureus Oxford, S. aureus WCUH29, S. pneumoniae 1629, S. pneumoniae N1387, S. pneumoniae ERY 2.**
Examples 1-5, 7, 12 and 14-16 have an MIC of less than 1µg/ml, and Examples 6, 8-11, 12 and 17 have an MIC of less than 8µg/ml against one or more of the above range of gram positive and gram negative bacteria.

## Claims

1. A compound of formula (I) or a pharmaceutically acceptable acid addition salt, quaternary ammonium salt, solvate and/or N-oxide thereof: 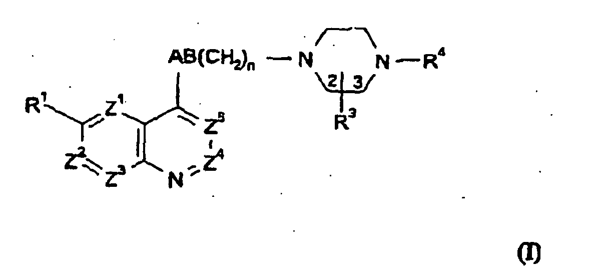 wherein:
one of Z¹, Z², Z³, Z⁴ and Z⁵ is N, one is CR^{1a} and the remainder are CH, or one of Z¹, Z², Z³, Z⁴ and Z⁵ is CR^{1a} and the remainder are CH;
or when Z⁵ is CR^{1a}, R^{1a} may instead be cyano, hydroxymethyl or carboxy;
R¹ and R^{1a} are independently selected from hydrogen; hydroxy; (C₁₋₆) alkoxy optionally substituted by (C₁₋₆)alkoxy, amino, piperidyl, guanidino or amidino any of which is optionally N-substituted by one or two (C₁₋₆)alkyl, acyl or (C₁₋₆)alkylsulphonyl groups, (C₁₋₆)alkylthio, heterocyclylthio, heterocyclyloxy, arylthio, aryloxy, acylthio, acyloxy or (C₁₋₆)alkylsulphonyloxy; (C₁₋₆)alkoxy-substituted (C₁₋₆)alkyl; halogen; (C₁₋₆)alkyl; (C₁₋₆)alkylthio; trifluoromethyl; nitro; azido; acyl; acyloxy; acylthio; (C₁₋₆)alkylsulphonyl; (C₁₋₆)alkylsulphoxide; arylsulphonyl; arylsulphoxide or an amino, piperidyl, guanidino or amidino group optionally N-substituted by one or two (C₁₋₆)alkyl, acyl or (C₁₋₆)alkylsulphonyl groups,
provided that when none of Z¹, Z², Z³, Z⁴ and Z⁵ is N, then R¹ is not hydrogen;
R³ is hydrogen; or
R³ is in the 2- or 3-position and is:
carboxy; (C₁₋₆)alkoxycarbonyl; aminocarbonyl wherein the amino group is optionally substituted by hydroxy, (C₁₋₆)alkyl, hydroxy(C₁₋₆)alkyl, aminocarbonyl(C₁₋₆)alkyl, (C₂₋₆)alkenyl, (C₁₋₆)alkylsulphonyl, trifluoromethylsulphonyl, (C₂₋₆)alkenylsulphonyl, (C₁₋₆)alkoxycarbonyl, (C₁₋₆)alkylcarbonyl, (C₂₋₆)alkenyloxycarbonyl or (C₂₋₆)alkenylcarbonyl and optionally further substituted by (C₁₋₆)alkyl, hydroxy(C₁₋₆)alkyl, aminocarbonyl(C₁₋₆)alkyl or (C₂₋₆)alkenyl; cyano; tetrazolyl; 2-oxo-oxazolidinyl optionally substituted by R¹⁰; 3-hydroxy-3-cyclobutene-1,2-dione-4-yl; 2,4-thiazolidinedione-5-yl; tetrazol-5-ylaminocarbonyl; 1,2,4-triazol-5-yl optionally substituted by R¹⁰; or 5-oxo-1,2,4-oxadiazol-3-yl; or
(C₁₋₄)alkyl or ethenyl optionally substituted with any of the groups listed above for R³ and/or 0 to 2 groups R¹² independently selected from:
halogen; (C₁₋₆)alkylthio; trifluoromethyl; (C₁₋₆)alkoxycarbonyl; (C₁₋₆)alkylcarbonyl; (C₂₋₆)alkenyloxycarbonyl; (C₂₋₆)alkenylcarbonyl; hydroxy optionally substituted by (C ₁₋₆)alkyl, (C₂₋₆)alkenyl, (C ₁₋₆)alkoxycarbonyl, (C ₁₋₆)alkylcarbonyl, (C₂₋₆)alkenyloxycarbonyl, (C₂₋₆)alkenylcarbonyl or aminocarbonyl wherein the amino group is optionally substituted by (C ₁₋₆)alkyl, (C₂₋₆)alkenyl, (C ₁₋₆)alkylcarbonyl or (C₂₋₆)alkenylcarbonyl; amino optionally mono- or disubstituted by (C₁₋₆)alkoxycarbonyl, (C ₁₋₆)alkylcarbonyl, (C₂₋₆)alkenyloxycarbonyl, (C₂₋₆)alkenylcarbonyl, (C₁₋₆)alkyl, (C₂₋₆)alkenyl, (C ₁₋₆)alkylsulphonyl, (C₂₋₆)alkenylsulphonyl or aminocarbonyl wherein the amino group is optionally substituted by (C₁₋₆)alkyl or (C₂₋₆)alkenyl; aminocarbonyl wherein the amino group is optionally substituted by (C ₁₋₆)alkyl, hydroxy(C ₁₋₆)alkyl, aminocarbonyl(C ₁₋₆)alkyl, (C₂₋₆ )alkenyl, (C₁₋₆)alkoxycarbonyl, (C₁₋₆)alkylcarbonyl, (C₂₋₆)alkenyloxycarbonyl or (C₂₋₆)alkenylcarbonyl and optionally further substituted by (C₁₋₆)alkyl, hydroxy(C₁₋₆)alkyl, aminocarbonyl(C₁₋₆)alkyl or (C₂₋₆)alkenyl; oxo; (C₁₋₆)alkylsulphonyl; (C₂₋₆)alkenylsulphonyl; or (C ₁₋₆)aminosulphonyl wherein the amino group is optionally substituted by (C₁₋₆)alkyl or (C₂₋₆)alkenyl;
in addition when R³ is disubstituted with a hydroxy or amino containing substituent and a carboxy containing substituent these may optionally together form a cyclic ester or amide linkage, respectively;
R¹⁰ is selected from (C₁₋₄)alkyl; (C₂₋₄)alkenyl and aryl any of which may be optionally substituted by a group R¹² as defined above; carboxy; aminocarbonyl wherein the amino group is optionally substituted by hydroxy, (C₁₋₆)alkyl, (C₂₋₆)alkenyl, (C₁₋₆)alkylsulphonyl, trifluoromethylsulphonyl, (C₂₋₆)alkenylsulphonyl, (C₁₋₆)alkoxycarbonyl, (C₁₋₆)alkylcarbonyl, (C₂₋₆)alkenyloxycarbonyl or (C₂₋₆)alkenylcarbonyl; and
R⁴ is a group-V-X¹-X²-X³-X⁴ in which:
V is CH₂, CO or SO₂;
X¹ is CR¹⁴R¹⁵;
X² is NR¹³, O, SO₂ or CR¹⁴R¹⁵;
X³ is NR¹³,O or CR¹⁴R¹⁵; wherein:
each of R¹⁴ and R¹⁵ is independently selected from: hydrogen; (C₁₋₄)alkoxy; (C₁₋₄)alkylthio; trifluoromethyl; cyano; (C₁₋₄)alkyl; (C₂₋₄)alkenyl; (C₁₋₄)allcoxycarbonyl; (C₁₋₄)alkylcarbonyl; (C₂₋₄)alkenyloxycarbonyl; (C₂₋₄)allcenylcarbonyl; hydroxy, amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C₁₋₄)alkyl or (C₂₋₄)alkenyl, provided that R¹⁴ and R¹⁵ on the same carbon atom are not both selected from optionally substituted hydroxy and optionally substituted amino; or
R¹⁴ and R¹⁵ together represent oxo;
R¹³ is hydrogen; trifluoromethyl, (C₁₋₆)alkyl; (C₂₋₆)alkenyl; (C₁₋₆)alkoxycarbonyl; (C₁₋₆)alkylcarbonyl; aminocarbonyl wherein the amino group is optionally substituted by (C₁₋₆)alkoxycarbonyl, (C₁₋₆)alkylcarbonyl, (C₂₋₆ )alkenyloxycarbonyl, (C₂₋₆)alkenylcarbonyl, (C₁₋₆)alkyl or (C₂₋₆)alkenyl and optionally further substituted by (C₁₋₆)alkyl or (C₂₋₆)alkenyl;
two R¹⁴ groups or an R¹³ and an R¹⁴ group in X¹, X² and X³ together with the atoms to which they are attached and, if appropriate, the intervening group X² form a 5 or 6 membered carbocyclic or heterocyclic ring and the remaining R¹³, R¹⁴ and R¹⁵ groups are as above defined; or
two R¹⁴ groups or an R¹³ and an R¹⁴ group on adjacent atoms together represent a bond and the remaining R¹³, R¹⁴ and R ¹⁵ groups are as above defined;
X⁴ is phenyl or C or N linked monocyclic aromatic 5- or 6-membered heterocycle containing up to four heteroatoms selected from O, S and N and optionally C-substituted by up to three groups selected from (C₁₋₄)alkylthio; halo; carboxy(C₁₋₄)alkyl; halo(C₁₋₄)alkoxy; halo(C₁₋₄)alkyl; (C₁₋₄)alkyl; (C₂₋₄)alkenyl; (C₁₋₄ )alkoxycarbonyl; formyl; (C₁₋₄)alkylcarbonyl; (C₂₋₄)alkenyloxycarbonyl;(C₂₋₄)alkenylcarbonyl; (C₁₋₄)alkylcarbonyloxy; (C₁₋₄)allcoxycarbonyl(C₁₋₄)alkyl; hydroxy; hydroxy(C₁₋₄)alkyl; mercapto(C₁₋₄)alkyl; (C₁₋₄)alkoxy; nitro; cyano; carboxy; amino or aminocarbonyl optionally substituted as for corresponding substituents in R³; (C₁₋₄)alkylsulphonyl; (C₂₋₄)alkenylsulphonyl; or aminosulphonyl wherein the amino group is optionally substituted by (C ₁₋₄)alkyl or (C₂₋₄)alkenyl; aryl; aryl(C ₁₋₄)alkyl or aryl(C₁₋₄)alkoxy; and
optionally N substituted by trifluoromethyl; (C₁₋₄)alkyl optionally substituted by hydroxy, (C ₁₋₆)alkoxy, (C ₁₋₆)alkylthio, halo or trifluoromethyl; (C₂₋₄)alkenyl; aryl; aryl(C₁₋₄)alkyl; (C ₁₋₄)alkoxycarbonyl; (C₁₋₄)alkylcarbonyl; formyl; (C₁₋₆)alkylsulphonyl; or aminocarbonyl wherein the amino group is optionally substituted by (C₁₋₄)alkoxycarbonyl, (C ₁₋₄)alkylcarbonyl, (C₂₋₄)alkenyloxycarbonyl, (C₂₋₄)alkenylcarbonyl, (C₁₋₄)alkyl or (C₂₋₄)alkenyl and optionally further substituted by (C₁₋₄)alkyl or (C₂₋₄)alkenyl;
provided that when X⁴ is optionally substituted phenyl, -V-X¹-X²-X³- is not -(CH₂)₃O-, -CH₂-(C₃-alkenyl)- or -(CH₂)₃CO-;
n is 0 and AB is NR¹¹CO, CO-CR⁸R⁹, CR⁶R⁷-CO, NR¹¹SO₂, CR⁶R⁷-SO₂ or CR⁶R⁷⁻CR⁸R⁹, provided that R⁸ and R⁹ are not optionally substituted hydroxy or amino and R⁶ and R⁸ do not represent a bond:
or n is 1 and AB is NR¹¹CO, CO-CR⁸R⁹, CR⁶R⁷-CO, NR¹¹SO₂, CONR¹¹, CR⁶R⁷⁻CR⁸R⁹, O-CR⁸R⁹ or NR¹¹-CR⁸R⁹;
and wherein:
each of R⁶ and R⁷, R⁸ and R⁹ is independently selected from: H; (C₁₋₆)alkoxy; (C₁₋₆)alkylthio; halo; trifluoromethyl; azido; (C₁₋₆)alkyl; (C₂₋₆)alkenyl; (C₁₋₆)alkoxycarbonyl; (C ₁₋₆)alkylcarbonyl; (C₂₋₆)alkenyloxycarbonyl; (C₂₋₆)alkenylcarbonyl; hydroxy, amino or aminocarbonyl optionally substituted as for corresponding substituents in R³; (C ₁₋₆)alkylsulphonyl; (C₂₋₆)alkenylsulphonyl; or (C₁₋₆)aminosulphonyl wherein the amino group is optionally substituted by (C₁₋₆)alkyl or (C₂₋₆)alkenyl;
or R⁶ and R⁸ together represent a bond and R⁷ and R⁹ are as above defined;
and each R¹¹ is independently H; trifluoromethyl; (C₁₋₆)alkyl; (C₂₋₆)alkenyl; (C₁₋₆)alkoxycarbonyl; (C₁₋₆)alkylcarbonyl; or aminocarbonyl wherein the amino group is optionally substituted by (C ₁₋₆)alkoxycarbonyl, (C ₁₋₆)alkylcarbonyl, (C₂₋₆)alkenyloxycarbonyl, (C₂₋₆)alkenylcarbonyl, (C₁₋₆)alkyl or (C₂₋₆)alkenyl and optionally further substituted by (C ₁₋₆)alkyl or (C₂₋₆)alkenyl;
or where one of R³ and R⁶, R⁷, R⁸ or R⁹ contains a carboxy group and the other contains a hydroxy or amino group they may together form a cyclic ester or amide linkage.

2. A compound according to claim 1 wherein Z⁵ is CH or N, Z³ is CH or CF and Z¹, Z² and Z⁴ are each CH, or Z¹ is N, Z³ is CH or CF and Z², Z⁴ and Z⁵ are each CH.

3. A compound according to any preceding claim wherein R¹ is methoxy and R^{1a} is H or when Z³ is CR^{1a} it may be C-F.

4. A compound according to any preceding claim wherein R³ is hydrogen; CONH₂; 1-hydroxyalkyl; CH₂CO₂H; CH₂CONH₂; -CONHCH₂CONH₂; 1,2-dihydroxyalkyl; CH₂CN; 2-oxo-oxazolidin-5-yl or 2-oxo-oxazolidin-5-yl(C₁₋₄alkyl).

5. A compound according to any preceding claim wherein n is 0 and either A is CHOH and B is CH₂ or A is NH and B is CO.

6. A compound according to any preceding claim wherein V is CH₂ and -X¹-X²⁻X³- is -(CH₂)₂-O-, -CH₂-CH=CH-, -(CH₂)₃-, -(CH₂)₂-NH-, -CH(OH) -CH₂-NH-, - CH₂NHCO- or -CH₂CONH-.

7. A compound according to any preceding claim wherein X⁴ is 2-pyridyl, 3-fluorophenyl, 3,5-difluorophenyl or 1,3-thiazole-2-yl.

8. A compound according to claim 1 selected from:
*(R)*-1-(3,5-Difluoro-phenylamino)-3-{4-[*(R)*-2-hydroxy-2-(6-methoxy-quinolin-4-yl)-ethyl]-piperazin-1-yl}-propan-2-ol
*(R*/*S*)-1-(3,5-Difluoro-phenylamino)-3-{4-[*(R)*-2-hydroxy-2-(6-methoxy-quinotin-4-yl)-ethyl]-piperazin-1-yl}-propan-2-ol
3-(3,5-Difluoro-phenyl)-5-{4-[*(R)*-2-hydroxy-2-(6-methoxy-quinolin-4-yl)-ethyl]-piperazin-1-ylmethyl}-oxazolidin-2-one
*N*-(2-{4-[*(R)*-2-Hydroxy-2-(6-methoxy-quinolin-4-yl)-ethyl]-piperazin-1-yl}-ethyl)-benzamide
3,5-Difluoro-*N*-(2-{4-[*(R)*-2-hydroxy-2-(6-methoxy-quinolin-4-yl)-ethyl]-piperazin-1-yl}-ethyl)-benzamide
Pyridine-2-carboxylic acid (2-{4-[*(R)*-2-hydroxy-2-(6-methoxy-quinolin-4-yl)-ethyl]-piperazin-1-yl}-ethyl)-amide
Thiophene-2-carboxylic acid (2- {4-[*(R)*-2-hydroxy-2-(6-methoxy-quinolin-4-yl)-ethyl]-piperazin-1-yl}-ethyl)-amide
Furan-2-carboxylic acid (2- {4-[*(R)*-2-hydroxy-2-(6-methoxy-quinolin-4-yl)-ethyl]-piperazin- 1-yl}-ethyl)-amide
3-Cyano-*N*-(2-{4-[*(R)*-2-hydxoxy-2-(6-methoxy-quinolin-4-yl)-ethyl]-piperazin-1-yl}-ethyl)-benzamide
*N*-(2-{4-[*(R)*-2-Hydroxy-2-(6-methoxy-quinolin-4-yl)-ethyl]-piperazin-1-yl}-ethyl)-trifluoromethyl-benzamide
(E)-1-{4-[*(R)*-2-Hydroxy-2-(6-methoxy-quinolin-4-yl)-ethyl]-piperazin-1-yl}-4-phenyl-but-3-en-1-one
*(R)*-1-(6-Methoxy-quinolin-4-yl)-2-[4-(4-phenyl-butyl)-piperazin-1-yl]-ethanol
*(S)*-1-(6-Methoxy-quinolin-4-yl)-2-[4-(4-phenyl-butyl)-piperazin-1-yl]-ethylamine
*N*-(3,5-Difluoro-phenyl)-3-{4-[*(R)*-2-hydroxy-2-(6-methoxy-quinohn-4-yl)-ethyl]-piperazin-1-yl}-propionamide
1-{4-[*(R)*-2-Hydroxy-2-(6-methoxy-quinolin-4-yl)-ethyl]-piperazin-1-yl}-3-phenoxy-propan-2-ol
3-(3-Fluoro-phenyl)-5-{4-[*(R)*-2-hydroxy-2-(6-methoxy-quinolin-4-yl)-ethyl]-piperazin -1-ylmethyl}-oxazolidin-2-one and
*(S)*-1-{4-[*(R)*-2-Hydroxy-2-(6-methoxy-quinolin-4-yl)-ethyl]-piperazin-1-yl}-3-phenylamino-propan-2-ol
or a pharmaceutically acceptable acid addition salt, quaternary ammonium salt, solvate and/or N-oxide thereof.

9. The use of a compound according to claim 1, in the manufacture of a medicament for use in the treatment of bacterial infections in mammals.

10. A pharmaceutical composition comprising a compound according to claim 1, and a pharmaceutically acceptable carrier.

11. A process for preparing a compound according to claim 1, which process comprises reacting a compound of formula (IV) with a compound of formula (V): 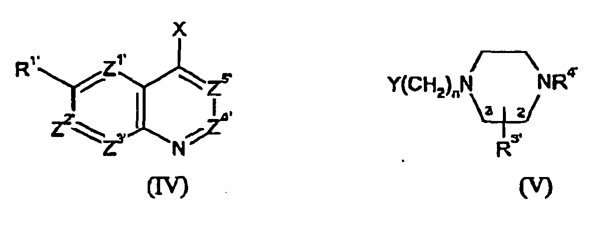 wherein n is as defined in formula (I); Z^{1'}, Z^{2'}, Z^{3'}, Z^{4'}, Z^{5'}, R^{1'}, R^{3'} and R^{4'} are Z¹, Z², Z³, Z⁴, Z⁵, R¹, R³ and R⁴ as defined in formula (I) or groups convertible thereto; and X and Y may be the following combinations:
(i) X is A'-COW, Y is H and n is 0;
(ii) X is CR⁶=CR⁸R⁹, Y is H and n is 0;
(iii) X is oxirane, Y is H and n is 0;
(iv) X is N=C=O and Y is H and n is 0;
(v) one of X and Y is CO₂R^{y} and the other is CH₂CO₂R^{x};
(vi) X is CHR⁶R⁷ and Y is C(=O)R⁸;
(vii) X is CR⁷=PR^{z}₃ and Y is C(=O)R⁹ and n=1;
(viii) X is C(=O)R⁷ and Y is CR⁹=PR^{z}₃ and n=1;
(ix) Y is COW and X is NHR^{11'} or NR^{11'}COW and n=0 or 1 or when n=1 X is COW and Y is NHR^{11'}, NCO or NR^{11'}COW;
(x) X is NHR^{11'} and Y is C(=O)R⁸ and n=1;
(xi) X is NHR^{11'} and Y is CR⁸R⁹W and n=1;
(xii) X is NR^{11'}COCH₂W or NR^{11'}SO₂CH₂W and Y is H and n=0;
(xiii) X is CR⁶R⁷SO₂W and Y is H and n=0;
(xiv) X is W or OH and Y is CH₂OH and n is 1;
(xv) X is NHR^{11'} and Y is SO₂W or X is NR^{11'}SO₂W and Y is H, and n is 0;
in which W is a leaving group, e.g. halo or imidazolyl; R^{x} and R^{y} are (C₁₋₆)alkyl; R^{z} is aryl or (C₁₋₆)alkyl; A' and NR^{11'} are A and NR¹¹ as defined in formula (I), or groups convertible thereto; and oxirane is: 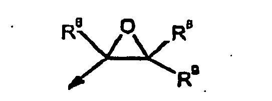 wherein R⁶,R⁸ and R⁹ are as defined in formula (I);
and thereafter optionally or as necessary converting A', Z^{1'}, Z^{2'}, Z^{3'}, Z^{4'}, Z^{5'}, R^{1'}, R^{3'},
R^{4'} and NR^{11'} to A, Z¹, Z² Z³, Z⁴, Z⁵, R¹, R³, R⁴ and NR¹¹; converting A-B to other A-B, interconverting R¹, R³ and/or R⁴, and/or forming a pharmaceutically acceptable acid addition salt, quaternary ammonium salt, solvate and/or N-oxide thereof.

## Patentansprüche

1. Verbindung der Formel (I) oder ein pharmazeutisch verträgliches Säureadditionssalz, quartäres Ammoniumsalz, Solvat und/oder N-Oxid davon: 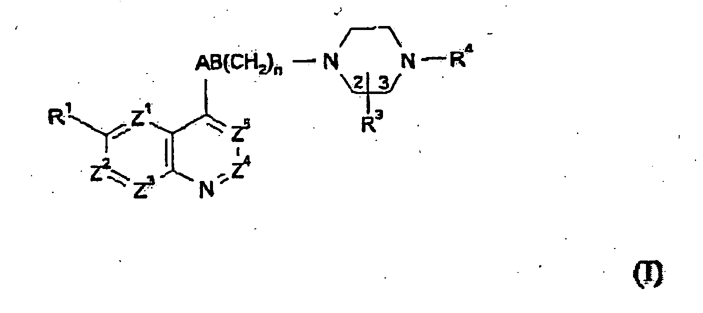 wobei:
einer der Reste Z¹, Z², Z³; Z⁴ und Z⁵ gleich N ist, einer CR^{1a} ist und die restlichen CH sind, oder einer der Reste Z¹, Z², Z³, Z⁴ und Z⁵ gleich CR^{1a} ist und die restlichen CH sind; oder wenn Z⁵ gleich CR^{1a} ist, R^{1a} statt dessen Cyano, Hydroxymethyl oder Carboxy sein kann;
R¹ und R^{1a} unabhängig ausgewählt sind aus Wasserstoff; Hydroxy; (C₁₋₆)-Alkoxy, gegebenenfalls substituiert mit (C₁₋₆)-Alkoxy, Amino, Piperidyl, Guanidino oder Amidino, von denen jedes gegebenenfalls N-substituiert ist mit einem oder zwei (C₁₋₆)-Alkyl-, Acyl- oder (C₁₋₆)-Alkylsulfonylresten, (C₁₋₆)-Alkylthio, Heterocyclylthio, Heterocyclyloxy, Arylthio, Aryloxy, Acylthio, Acyloxy oder (C₁₋₆)-Alkylsulfonyloxy; (C₁₋₆)-Alkoxy-substituiertem (C₁₋₆)-Alkyl; Halogen; (C₁₋₆)-Alkyl; (C₁₋₆)-Alkylthio; Trifluormethyl; Nitro; Azido; Acyl; Acyloxy; Acylthio; (C₁₋₆)-Alkylsulfonyl; (C₁₋₆)-Alkylsulfoxid; Arylsulfonyl; Arylsulfoxid oder einem Amino-, Piperidyl-, Guanidino- oder Amidinorest, gegebenenfalls N-substituiert mit einem oder zwei (C₁₋₆)-Alkyl-, Acyl- oder (C₁₋₆)-Alkylsulfonylresten,
mit der Maßgabe, dass, wenn keiner der Reste Z¹, Z², Z³, Z⁴ und Z⁵ gleich N ist, dann R¹ nicht Wasserstoff ist;
R³ Wasserstoff ist; oder
R³ in 2- oder 3-Position ist und bedeutet:
Carboxy; (C₁₋₆)-Alkoxycarbonyl; Aminocarbonyl, wobei die Aminogruppe gegebenenfalls substituiert ist mit Hydroxy, (C₁₋₆)-Alkyl, Hydroxy-(C₁₋₆)-alkyl, Aminocarbonyl-(C₁₋₆)-alkyl, (C₂₋₆)-Alkenyl, (C₁-₆)-Alkylsulfonyl, Trifluormethylsulfonyl, (C₂₋₆)-Alkenylsulfonyl, (C₁₋₆)-Alkoxycarbonyl, (C₁₋₆)-Alkylcarbonyl, (C₂₋₆)-Alkenyloxycarbonyl oder (C₂₋₆)-Alkenylcarbonyl und gegebenenfalls weiter substituiert ist mit (C₁₋₆)-Alkyl, Hydroxy-(C₁₋₆)-alkyl, Aminocarbonyl-(C₁₋₆)-alkyl oder (C₂₋₆)-Alkenyl; Cyano; Tetrazolyl; 2-Oxo-oxazolidinyl, gegebenenfalls substituiert mit R¹⁰; 3-Hydroxy-3-cyclobuten-1,2-dion-4-yl; 2,4-Thiazolidindion-5-yl; Tetrazol-5-ylaminocarbonyl; 1,2,4-Triazol-5-yl, gegebenenfalls substituiert mit R¹⁰; oder 5-Oxo-1,2,4-oxadiazol-3-yl; oder
(C₁₋₄)-Alkyl oder Ethenyl, gegebenenfalls substituiert mit einem der oben für R³ aufgeführten Reste und/oder 0 bis 2 Resten R¹², die unabhängig ausgewählt sind aus: Halogen; (C₁₋₆)-Alkylthio; Trifluormethyl; (C₁₋₆)-Alkoxycarbonyl; (C₁₋₆)-Alkylcarbonyl; (C₂₋₆)-Alkenyloxycarbonyl; (C₂₋₆)-Alkenylcarbonyl; Hydroxy, gegebenenfalls substituiert mit (C₁₋₆)-Alkyl, (C₂₋₆)-Alkenyl, (C₁₋₆)-Alkoxycarbonyl, (C₁₋₆)-Alkylcarbonyl, (C₂₋₆)-Alkenyloxycarbonyl, (C₂₋₆)-Alkenylcarbonyl oder Aminocarbonyl, wobei die Aminogruppe gegebenenfalls substituiert ist mit (C₁₋₆)-Alkyl, (C₂₋₆)-Alkenyl, (C₁₋₆)-Alkylcarbonyl oder (C₂₋₆)-Alkenylcarbonyl; Amino, gegebenenfalls mono- oder disubstituiert mit (C₁₋₆)-Alkoxycarbonyl, (C₁₋₆)-Alkylcarbonyl, (C₂₋₆)-Alkenyloxycarbonyl, (C₂₋₆)-Alkenylcarbonyl, (C₁₋₆)-Alkyl, (C₂₋₆)-Alkenyl, -(C₁₋₆)-Alkylsulfonyl, (C₂₋₆)-Alkenylsulfonyl oder Aminocarbonyl, wobei die Aminogruppe gegebenenfalls substituiert ist mit (C₁₋₆)-Alkyl oder (C₂₋₆)-Alkenyl; Aminocarbonyl, wobei die Aminogruppe gegebenenfalls substituiert ist mit (C₁₋₆)-Alkyl, Hydroxy-(C₁₋₆)-alkyl, Aminocarbonyl-(C₁₋₆)-alkyl, (C₂₋₆)-Alkenyl, (C₁₋₆)-Alkoxycarbonyl, (C₁₋₆)-Alkylcarbonyl, (C₂₋₆)-Alkenyloxycarbonyl oder (C₂₋₆)-Alkenylcarbonyl und gegebenenfalls weiter substituiert ist mit (C₁₋₆)-Alkyl, Hydroxy-(C₁₋₆)-alkyl, Aminocarbonyl-(C₁₋₆)-alkyl oder (C₂₋₆)-Alkenyl; Oxo; (C₁₋₆)-Alkylsulfonyl; (C₂₋₆)-Alkenylsulfonyl; oder (C₁₋₆)-Aminosulfonyl, wobei die Aminogruppe gegebenenfalls substituiert ist mit (C₁₋₆)-Alkyl oder (C₂₋₆)-Alkenyl;
zusätzlich, wenn R³ disubstituiert ist mit einem Hydroxy- oder Amino-enthaltenden Substituenten und einem Carboxy-enthaltenden Substituenten, diese gegebenenfalls zusammen eine cyclische Ester- bzw. Amidbindung bilden können;
R¹⁰ ausgewählt ist aus (C₁₋₄)-Alkyl; (C₂₋₄)-Alkenyl und Aryl, von denen jedes gegebenenfalls substituiert sein kann mit einem wie vorstehend definierten Rest R¹²; Carboxy; Aminocarbonyl, wobei die Aminogruppe gegebenenfalls substituiert ist mit Hydroxy, (C₁₋₆)-Alkyl, (C₂₋₆)-Alkenyl, (C₁₋₆)-Alkylsulfonyl, Trifluormethylsulfonyl, (C₂₋₆)-Alkenylsulfonyl, (C₁₋₆)-Alkoxycarbonyl, (C₁₋₆)-Alkylcarbonyl, (C₂₋₆)-Alkenyloxycarbonyl oder (C₂₋₆)-Alkenylcarbonyl; und
R⁴ ein Rest -V-X¹-X²-X³-X⁴ ist, wobei:
V gleich CH₂, CO oder SO₂ ist;
X¹ gleich CR¹⁴R¹⁵ ist;
X² gleich NR¹³, O, SO₂ oder CR¹⁴R¹⁵ ist;
X³ gleich NR¹³, O oder CR¹⁴R¹⁵ ist; wobei:
jeder der Reste R¹⁴ und R¹⁵ unabhängig ausgewählt ist aus: Wasserstoff; (C₁₋₄)-Alkoxy; (C₁₋₄)-Alkylthio; Trifluormethyl; Cyano; (C₁₋₄)-Alkyl; (C₂₋₄)-Alkenyl; (C₁₋₄)-Alkoxycarbonyl; (C₁₋₄)-Alkylcarbonyl; (C₂₋₄)-Alkenyloxycarbonyl; (C₂₋₄)-Alkenylcarbonyl; Hydroxy, Amino oder Aminocarbonyl, gegebenenfalls substituiert wie für die entsprechenden Substituenten in R³; (C₁₋₄)-Alkylsulfonyl; (C₂₋₄)-Alkenylsulfonyl; oder Aminosulfonyl, wobei die Aminogruppe gegebenenfalls substituiert ist mit (C₁₋₄)-Alkyl oder (C₂₋₄)-Alkenyl, mit der Maßgabe, dass R¹⁴ und R¹⁵ an dem gleichen Kohlenstoffatom nicht beide ausgewählt sind aus gegebenenfalls substituiertem Hydroxy und gegebenenfalls substituiertem Amino; oder
R¹⁴ und R¹⁵ zusammen Oxo bedeuten;
R¹³ Wasserstoff; Trifluormethyl, (C₁₋₆)-Alkyl; (C₂₋₆)-Alkenyl; (C₁₋₆)-Alkoxycarbonyl; (C₁₋₆)-Alkylcarbonyl; Aminocarbonyl, wobei die Aminogruppe gegebenenfalls substituiert ist mit (C₁₋₆)-Alkoxycarbonyl, (C₁₋₆)-Alkylcarbonyl, (C₂₋₆)-Alkenyloxycarbonyl, (C₂₋₆)-Alkenylcarbonyl, (C₁₋₆)-Alkyl oder (C₂₋₆)-Alkenyl und gegebenenfalls weiter substituiert ist mit (C₁₋₆)-Alkyl oder (C₂₋₆)-Alkenyl, ist;
zwei Reste R¹⁴ oder ein Rest R¹³ und ein Rest R¹⁴ in X¹, X² und X³ zusammen mit den Atomen, an die sie gebunden sind, und, falls passend, dem dazwischenliegenden Rest X² einen 5- oder 6-gliedrigen carbocyclischen oder heterocyclischen Ring bilden und die restlichen Reste R¹³, R¹⁴ und R¹⁵ wie vorstehend definiert sind; oder
zwei Reste R¹⁴ oder ein Rest R¹³ und ein Rest R¹⁴ an benachbarten Atomen zusammen eine Bindung darstellen und die restlichen Reste R¹³, R¹⁴ und R¹⁵ wie vorstehend definiert sind;
X⁴ Phenyl oder ein C- oder N-gebundener, monocyclischer, aromatischer 5- oder 6-gliedriger Heterocyclus mit bis zu 4 Heteroatomen, ausgewählt aus O, S und N, und gegebenenfalls C-substituiert ist mit bis zu drei Resten, ausgewählt aus (C₁₋₄)-Alkylthio; Halogen; Carboxy-(C₁₋₄)-alkyl; Halogen-(C₁₋₄)-alkoxy; Halogen-(C₁₋₄)-alkyl; (C₁₋₄)-Alkyl; (C₂₋₄)-Alkenyl; (C₁₋₄)-Alkoxycarbonyl; Formyl; (C₁₋₄)-Alkylcarbonyl; (C₂₋₄)-Alkenyloxycarbonyl; (C₂₋₄)-Alkenylcarbonyl,; (C₁₋₄)-Alkylcarbonyloxy; (C₁₋₄)-Alkoxycarbonyl-(C₁₋₄)-alkyl; Hydroxy; Hydroxy-(C₁₋₄)-alkyl; Mercapto-(C₁₋₄)-alkyl; (C₁₋₄)-Alkoxy; Nitro; Cyano; Carboxy; Amino oder Aminocarbonyl, gegebenenfalls substituiert wie für entsprechende Substituenten in R³; (C₁₋₄)-Alkylsulfonyl; (C₂₋₄)-Alkenylsulfonyl; oder Aminosulfonyl, wobei die Aminogruppe gegebenenfalls substituiert ist mit (C₁₋₄)-Alkyl oder (C₂₋₄)-Alkenyl; Aryl; Aryl-(C₁₋₄)-alkyl oder Aryl-(C₁₋₄)-alkoxy; und
gegebenenfalls N-substituiert ist mit Trifluormethyl; (C₁₋₄)-Alkyl, gegebenenfalls substituiert mit Hydroxy, (C₁₋₆)-Alkoxy, (C₁₋₆)-Alkylthio, Halogen oder Trifluormethyl; (C₂₋₄)-Alkenyl; Aryl; Aryl-(C₁₋₄)-alkyl; (C₁₋₄)-Alkoxycarbonyl; (C₁₋₄)-Alkylcarbonyl; Formyl; (C₁₋₆)-Alkylsulfonyl; oder Aminocarbonyl, wobei die Aminogruppe gegebenenfalls substituiert ist mit (C₁₋₄)-Alkoxycarbonyl, (C₁₋₄)-Alkylcarbonyl, (C₂₋₄)-Alkenyloxycarbonyl, (C₂₋₄)-Alkenylcarbonyl, (C₁₋₄)-Alkyl oder (C₂₋₄)-Alkenyl und gegebenenfalls weiter substituiert ist mit (C₁₋₄)-Alkyl oder (C₂₋₄)-Alkenyl;
mit der Maßgabe, dass, wenn X⁴ gegebenenfalls substituiertes Phenyl ist, -V-X¹-X²-X³⁻nicht -(CH₂)₃O-, -CH₂-(C₃-Alkenyl)- oder -(CH₂)₃CO- ist;
n gleich 0 ist und AB gleich NR¹¹CO, CO-CR⁸R⁹, CR⁶R⁷-CO, NR¹¹SO₂, CR⁶R⁷-SO₂ oder CR⁶R⁷-CR⁸R⁹ ist, mit der Maßgabe, dass R⁸ und R⁹ nicht gegebenenfalls substituiertes Hydroxy oder Amino sind und R⁶ und R⁸ keine Bindung bedeuten;
oder n gleich 1 ist und AB gleich NR¹¹CO, CO-CR⁸R⁹, CR⁶R⁷-CO, NR¹¹SO₂, CONR¹¹, CR⁶R⁷-CR⁸R⁹ O-CR⁸R⁹ oder NR¹¹-CR⁸R⁹ ist;
und wobei:
jeder der Reste R⁶ und R⁷, R⁸ und R⁹ unabhängig ausgewählt ist aus: H; (C₁₋₆)-Alkoxy; (C₁₋₆)-Alkylthio; Halogen; Trifluormethyl; Azido; (C₁₋₆)-Alkyl; (C₂₋₆)-Alkenyl; (C₁₋₆)-Alkoxycarbonyl; (C₁₋₆)-Alkylcarbonyl; (C₂₋₆)-Alkenyloxycarbonyl; (C₂₋₆)-Alkenylcarbonyl; Hydroxy, Amino oder Aminocarbonyl, gegebenenfalls substituiert wie für entsprechende Substituenten in R³; (C₁₋₆)-Alkylsulfonyl; (C₂₋₆)-Alkenylsulfonyl; oder (C₁₋₆)-Aminosulfonyl, wobei die Aminogruppe gegebenenfalls substituiert ist mit (C₁₋₆)-Alkyl oder (C₂₋₆)-Alkenyl;
oder R⁶ und R⁸ zusammen eine Bindung darstellen und R⁷ und R⁹ wie vorstehend definiert sind;
und jeder Rest R¹¹ unabhängig H; Trifluormethyl; (C₁₋₆)-Alkyl; (C₂₋₆)-Alkenyl; (C₁₋₆)-Alkoxycarbonyl; (C₁₋₆)-Alkylcarbonyl; oder Aminocarbonyl ist, wobei die Aminogruppe gegebenenfalls substituiert ist mit (C₁₋₆)-Alkoxycarbonyl, (C₁₋₆)-Alkylcarbonyl, (C₂₋₆)-Alkenyloxycarbonyl, (C₂₋₆)-Alkenylcarbonyl, (C₁₋₆)-Alkyl oder (C₂₋₆)-Alkenyl und gegebenenfalls weiter substituiert ist mit (C₁₋₆)-Alkyl oder (C₂₋₆)-Alkenyl;
oder wenn einer der Reste R³ und R⁶, R⁷, R⁸ oder R⁹ eine Carboxygruppe enthält und der andere eine Hydroxy- oder Aminogruppe enthält, diese zusammen eine cyclische Ester- oder Amidbindung bilden können.

2. Verbindung nach Anspruch 1, wobei Z⁵ gleich CH oder N ist, Z³ gleich CH oder CF ist und Z¹, Z² und Z⁴ jeweils CH sind, oder Z¹ gleich N ist, Z³ gleich CH oder CF ist und Z², Z⁴ und Z⁵ jeweils CH sind.

3. Verbindung nach einem vorhergehenden Anspruch, wobei R¹ Methoxy ist und R^{1a} gleich H ist oder wenn Z³ gleich CR^{1a} ist, es C-F sein kann.

4. Verbindung nach einem vorhergehenden Anspruch, wobei R³ Wasserstoff; CONH₂; 1-Hydroxyalkyl; CH₂CO₂H; CH₂CONH₂; -CONHCH₂CONH₂; 1,2-Dihydroxyalkyl; CH₂CN; 2-Oxo-oxazolidin-5-yl oder 2-Oxo-oxazolidin-5-yl-(C₁₋₄-alkyl) ist.

5. Verbindung nach einem vorhergehenden Anspruch, wobei n gleich 0 ist und entweder A gleich CHOH ist und B gleich CH₂ ist oder A gleich NH ist und B gleich CO ist.

6. Verbindung nach einem vorhergehenden Anspruch, wobei V gleich CH₂ ist und -X¹-X²⁻X³- gleich -(CH₂)₂-O-, -CH₂-CH=CH-, -CH₂)₃-, -(CH₂)₂-NH-, -CH(OH-CH₂₋NH-, -CH₂NHCO- oder -CH₂CONH- ist.

7. Verbindung nach einem vorhergehenden Anspruch, wobei X⁴ 2-Pyridyl, 3-Fluorphenyl, 3,5-Difluorphenyl oder 1,3-Thiazol-2-yl ist.

8. Verbindung nach Anspruch 1, ausgewählt aus:
(R)-1-(3,5-Difluor-phenylamino)-3-{4-[(R)-2-hydroxy-2-(6-methoxy-chinolin-4-yl)-ethyl]-piperazin-1-yl}-propan-2-ol;
(R/S)-1-(3,5-Difluor-phenylamino)-3-{4-[(R)-2-hydroxy-2-(6-methoxy-chinolin-4-yl)-ethyl]-piperazin-1-yl}-propan-2-ol;
3-(3,5-Difluor-phenyl)-5-{4-[(R)-2-hydroxy-2-(6-methoxy-chiriolin-4-yl)-ethyl]-piperazin-1-ylmethyl}-oxazolidin-2-on;
N-(2- {4-[(R)-2-Hydroxy-2-(6-methoxy-chinolin-4-yl)-ethyl]-piperazin-1-yl}-ethyl)-benzamid;
3,5-Difluor-N-(2- {4-[(R)-2-hydroxy-2-(6-methoxy-chinolin-4-yl)-ethyl]-piperazin-1-yl}-ethyl)-benzamid;
Pyridin-2-carbonsäure-(2-{4-[(R)-2-hydroxy-2-(6-methoxy-chinolin-4-yl)-ethyl]-piperazin-1-yl}-ethyl)-amid;
Thiophen-2-carbonsäure-(2-{4-[(R)-2-hydroxy-2-(6-methoxy-chinolin-4-yl)-ethyl]-piperazin-1-yl}-ethyl)-amid;
Furan-2-carbonsäure-(2-{4-[(R)-2-hydroxy-2-(6-methoxy-chinolin-4-yl)-ethyl]-piperazin-1-yl}-ethyl)-amid;
3-Cyano-N-(2-{4-[(R)-2-hydroxy-2-(6-methoxy-chinolin-4-yl)-ethyl]-piperazin-1-yl}-ethyl)-benzamid;
N-(2- {4-[(R)-2-Hydroxy-2-(6-methoxy-chinolin-4-yl)-ethyl]-piperazin-1-yl} -ethyl)-trifluormethyl-benzamid;
(E)-1- {4-[(R)-2-Hydroxy-2-(6-methoxy-chinolin-4-yl)-ethyl]-piperazin-1-yl} -4-phenyl-but-3-en-1-on;
(R)-1-(6-Methoxy-chinolin-4-yl)-2-[4-(4-phenyl-butyl)-piperazin-1-yl]-ethanol;
(S)-1-(6-Methoxy-chinolin-4-yl)-2-[4-(4-phenyl-butyl)-piperazin-1-yl]-ethylamin;
N-(3,5-Difluor-phenyl)-3- {4-[(R)-2-hydroxy-2-(6-methoxy-chinolin-4-yl)-ethyl]-piperazin-1-yl}-propionamid;
1-{4-[(R)-2-Hydroxy-2-(6-methoxy-chinolin-4-yl)-ethyl]-piperazin-1-yl}-3-phenoxy-propan-2-ol;
3-(3-Fluor-phenyl)-5- {4-[(R)-2-hydroxy-2-(6-methoxy-chinolin-4-yl)-ethyl]-piperazin-1-ylmethyl}-oxazolidin-2-on und
(S)-1- {4-[(R)-2-Hydroxy-2-(6-methoxy-chinolin-4-yl)-ethyl]-piperazin-1-yl }-3-phenylamino-propan-2-ol
oder ein pharmazeutisch verträgliches Säureadditionssalz, quartäres Ammoniumsalz, Solvat und/oder N-Oxid davon.

9. Verwendung einer Verbindung gemäß Anspruch 1 bei der Herstellung eines Medikaments zur Verwendung bei der Behandlung bakterieller Infektionen bei Säugern.

10. Arzneimittel, umfassend eine Verbindung gemäß Anspruch 1 und einen pharmazeutisch verträglichen Träger.

11. Verfahren zur Herstellung einer Verbindung gemäß Anspruch 1, wobei das Verfahren das Umsetzen einer Verbindung der Formel (IV) mit einer Verbindung der Formel (V): 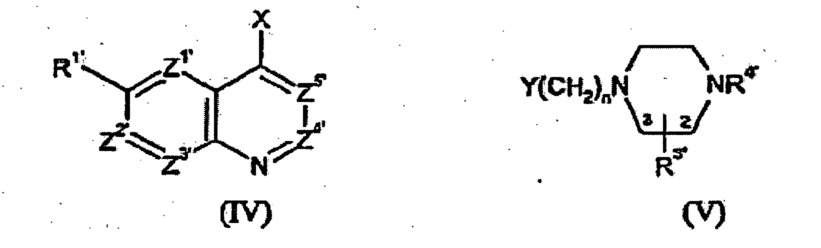 wobei n wie in Formel (I) definiert ist; Z^{1'}, Z^{2'}, Z^{3'}, Z^{4'}, Z^{5'}, R^{1'}, R^{3'} und R^{4'} gleich Z¹, Z², Z³, Z⁴, Z⁵, R¹, R³ und R⁴ wie in Formel (I) definiert oder dazu umwandelbare Reste sind; und X und Y die folgenden Kombinationen sein können:
(i) X ist A'-COW; Y ist H und n ist 0;
(ii) X ist CR⁶=CR⁸R⁹, Y ist H und n ist 0;
(iii) X ist Oxiran, Y ist H und n ist 0;
(iv) X ist N=C=O und Y ist H und n ist 0;
(v) einer der Reste X und Y ist CO₂R^{y} und der andere ist CH₂CO₂R^{x};
(vi) X ist CHR⁶R⁷ und Y ist C(=O)R⁸;
(vii) X ist CR⁷=PR^{z}₃ und Y ist C(=O)R⁹ und n=1 ;
(viii) X ist C(=O)R⁷ und Y ist CR⁹=PR^{z}₃ und n=1;
(ix) Y ist COW und X ist NHR^{11'} oder NR^{11'}COW und n=0 oder 1 oder wenn n=1, X ist COW und Y ist NHR^{11'}, NCO oder NR^{11'}COW;
(x) X ist NHR^{11'} und Y ist C(=O)R⁸ und n=1;
(xi) X ist NHR^{11'} und Y ist CR⁸R⁹W und n=1;
(xii) X ist NR^{11'}COCH₂W oder NR^{11'}SO₂CH₂W und Y ist H und n=0;
(xiii) X ist CR⁶R⁷SO₂W und Y ist H und n=0;
(xiv) X ist W oder OH und Y ist CH₂OH und n ist 1;
(xv) X ist NHR^{11'} und Y ist SO₂W oder X ist NR^{11'}SO₂W und Y ist H und n ist 0;
wobei W eine Abgangsgruppe, z.B. Halogen oder Imidazolyl ist; R^{x} und R^{y} (C₁₋₆)-Alkyl sind; R^{z} Aryl oder (C₁₋₆)-Alkyl ist; A' und NR^{11'} gleich A und NR¹¹ wie in Formel (I) definiert oder dazu umwandelbare Reste sind; und Oxiran ist: 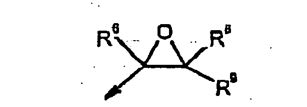 wobei R⁶, R⁸ und R⁹ wie in Formel (I) definiert sind;
und danach gegebenenfalls oder falls erforderlich Umwandeln von A', Z^{1'}, Z^{2'}, Z^{3'}, Z^{4'}, Z^{5'}, R^{1'}, R^{3'}, R^{4'} und NR^{11'} in A, Z¹, Z², Z³, Z⁴, Z⁵, R¹, R³, R⁴ und NR¹¹; Umwandeln von A-B in anderes A-B, Ineinanderumwandeln von R¹, R³ und/oder R⁴, und/oder Bilden eines pharmazeutisch verträglichen Säureadditionssalzes, quartären Ammoniumsalzes, Solvats und/oder N-Oxids davon umfasst.

## Revendications

1. Composé de formule (I) ou un sel d'addition d'acide, sel d'ammonium quaternaire, produit de solvatation et/ou N-oxyde pharmaceutiquement acceptable de ce composé : 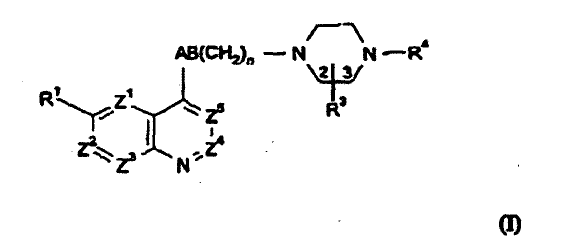 où :
l'un de Z¹, Z², Z³, Z⁴ et Z⁵ est N, l'un est CR^{1a} et les autres sont CH, ou bien l'un de Z¹, Z², Z³, Z⁴ et Z⁵ est CR^{1a} et les autres sont CH ;
ou, lorsque Z⁵ est CR^{1a}, R^{1a} peut être alors un groupe cyano, hydroxyméthyle ou carboxy ;
R¹ et R^{1a} sont choisis indépendamment parmi l'hydrogène ; un groupe hydroxy ; alkoxy en C₁ à C₆ facultativement substitué par un groupe alkoxy en C₁ à C₆, amino, pipéridyle, guanidino ou amidino pouvant chacun être facultativement substitué sur N par un ou deux groupes alkyle en C₁ à C₆, acyle ou (alkyle en C₁ à C₆)sulfonyle, (alkyle en C₁ à C₆)thio, hétérocyclylthio, hétérocyclyloxy, arylthio, aryloxy, acylthio, acyloxy ou (alkyle en C₁ à C₆)sulfonyloxy ; alkyle en C₁ à C₆ substitué par alkoxy en C₁ à C₆ ; halogène ; alkyle en C₁ à C₆ ; (alkyle en C₁ à C₆)thio ; trifluorométhyle ; nitro ; azido ; acyle ; acyloxy ; acylthio ; (alkyle en C₁ à C₆)sulfonyle ; (alkyle en C₁ à C₆)sulfoxyde ; arylsulfonyle ; arylsulfoxyde ou un groupe amino, pipéridyle, guanidino ou amidino facultativement substitué sur N par un ou deux groupes alkyle en C₁ à C₆, acyle ou (alkyle en C₁ à C₆) sulfonyle,
à condition que lorsque aucun de Z¹, Z², Z³, Z⁴ et Z⁵ n'est N, alors R¹ ne soit pas l'hydrogène ;
R³ est l'hydrogène ; ou bien
R³ est à la position 2 ou 3 et est :
un groupe carboxy ; (alkoxy en C₁ à C₆)carbonyle ; aminocarbonyle dans lequel le groupe amino est facultativement substitué par un groupe hydroxy, alkyle en C₁ à C₆, hydroxy(alkyle en C₁ à C₆), aminocarbonyl(alkyle en C₁ à C₆), alcényle en C₂ à C₆, (alkyle en C₁ à C₆)sulfonyle, trifluorométhylsulfonyle, (alcényle en C₂ à C₆)sulfonyle, (alkoxy en C₁ à C₆)carbonyle, (alkyle en C₁ à C₆)carbonyle, (alcényle en C₂ à C₆)oxycarbonyle ou (alcényle en C₂ à C₆)carbonyle et facultativement encore substitué par un groupe alkyle en C₁ à C₆, hydroxy(alkyle en C₁ à C₆), aminocarbonyl(alkyle en C₁ à C₆) ou alcényle en C₂ à C₆ ; cyano ; tétrazolyle ; 2-oxo-oxazolidinyle facultativement substitué par R¹⁰ ; 3-hydroxy-3-cyclobutène-1,2-dione-4-yle ; 2,4-thiazolidinedione-5-yle ; tétrazole-5-ylamino-carbonyle ; 1,2,4-triazole-5-yle facultativement substitué par R¹⁰ ; ou 5-oxo-1,2,4-oxadiazole-3-yle ; ou
un groupe alkyle en C₁ à C₄ ou éthényle facultativement substitué par n'importe lesquels des groupes cités ci-dessus pour R³ et/ou 0 à 2 groupes R¹² choisis indépendamment parmi :
un halogène ; un groupe (alkyle en C₁ à C₆) thio ; trifluorométhyle ; (alkoxy en C₁ à C₆)carbonyle ; (alkyle en C₁ à C₆) carbonyle ; (alcényle en C₂ à C₆) oxycarbonyle ; (alcényle en C₂ à C₆) carbonyle ; hydroxy facultativement substitué par un groupe alkyle en C₁ à C₆, alcényle en C₂ à C₆, (alkoxy en C₁ à C₆) carbonyle, (alkyle en C₁ à C₆) - carbonyle, (alcényle en C₂ à C₆) oxycarbonyle, (alcényle en C₂ à C₆)carbonyle ou aminocarbonyle dans lequel le groupe amino est facultativement substitué par un groupe alkyle en C₁ à C₆, alcényle en C₂ à C₆, (alkyle en C₁ à C₆) carbonyle ou (alcényle en C₂ à C₆)carbonyle ; amino facultativement mono- ou disubstitué par (alkoxy en C₁ à C₆)carbonyle, (alkyle en C₁ à C₆)carbonyle, (alcényle en C₂ à C₆)oxycarbonyle, (alcényle en C₂ à C₆)carbonyle, alkyle en C₁ à C₆, alcényle en C₂ à C₆, (alkyle en C₁ à C₆) sulfonyle, (alcényle en C₂ à C₆)sulfonyle ou aminocarbonyle dans lequel le groupe amino est facultativement substitué par un groupe alkyle en C₁ à C₆ ou alcényle en C₂ à C₆ ; aminocarbonyle dans lequel le groupe amino est facultativement substitué par un groupe alkyle en C₁ à C₆, hydroxy(alkyle en C₁ à C₆), aminocarbonyl(alkyle en C₁ à C₆), alcényle en C₂ à C₆, (alkoxy en C₁ à C₆) carbonyle, (alkyle en C₁ à C₆) carbonyle, (alcényle en C₂ à C₆) oxycarbonyle ou (alcényle en C₂ à C₆) carbonyle et facultativement encore substitué par un groupe alkyle en C₁ à C₆, hydroxy(alkyle en C₁ à C₆), aminocarbonyl(alkyle en C₁ à C₆) ou alcényle en C₂ à C₆ ; oxo ; (alkyle en C₁ à C₆) sulfonyle ; (alcényle en C₂ à C₆) sulfonyle ; ou aminosulfonyle en C₁ à C₆ dans lequel le groupe amino est facultativement substitué par un groupe alkyle en C₁ à C₆ ou alcényle en C₂ à C₆ ;
de plus, lorsque R³ est disubstitué avec un substituant contenant un groupe hydroxy ou amino et un substituant contenant un groupe carboxy, ceux-ci peuvent facultativement former ensemble une liaison ester ou amide cyclique, respectivement ;
R¹⁰ est choisi parmi un groupe alkyle en C₁ à C₄ ; alcényle en C₂ à C₄ et aryle pouvant chacun être facultativement substitué par un groupe R¹² tel que défini ci-dessus ; carboxy ; aminocarbonyle dans lequel le groupe amino est facultativement substitué par un groupe hydroxy, alkyle en C₁ à C₆, alcényle en C₂ à C₆, (alkyle en C₁ à C₆)sulfonyle, trifluorométhylsulfonyle, (alcényle en C₂ à C₆)sulfonyle, (alkoxy en C₁ à C₆)carbonyle, (alkyle en C₁ à C₆) carbonyle, (alcényle en C₂ à C₆) oxycarbonyle ou (alcényle en C₂ à C₆) carbonyle ; et
R⁴ est un groupe -V-X¹-X²-X³-X⁴ dans lequel :
V est CH₂, CO ou SO₂ ;
X¹ est CR¹⁴R¹⁵ ;
X² est NR¹³, O, SO₂ ou CR¹⁴R¹⁵ ;
X³ est NR¹³, O ou CR¹⁴R¹⁵ ; où :
chacun de R¹⁴ et R¹⁵ est choisi indépendamment parmi : l'hydrogène ; un groupe alkoxy en C₁ à C₄ ; (alkyle en C₁ à C₄)thio ; trifluorométhyle ; cyano ; alkyle en C₁ à C₄ ; alcényle en C₂ à C₄ ; (alkoxy en C₁ à C₄)carbonyle ; (alkyle en C₁ à C₄)carbonyle ; (alcényle en C₂ à C₄)oxycarbonyle ; (alcényle en C₂ à C₄)carbonyle ; hydroxy, amino ou aminocarbonyle facultativement substitué comme pour les substituants correspondants dans R³ ; (alkyle en C₁ à C₄) - sulfonyle ; (alcényle en C₂ à C₄)sulfonyle ; ou aminosulfonyle dans lequel le groupe amino est facultativement substitué par un groupe alkyle en C₁ à C₄ ou alcényle en C₂ à C₄, à condition que R¹⁴ et R¹⁵ présents sur le même atome de carbone ne soient pas tous deux choisis parmi un groupe hydroxy facultativement substitué ou amino facultativement substitué ; ou bien
R¹⁴ et R¹⁵ représentent ensemble un groupe oxo ;
R¹³ est l'hydrogène ; un groupe trifluorométhyle ; alkyle en C₁ à C₆ ; alcényle en C₂ à C₆ ; (alkoxy en C₁ à C₆)carbonyle ; (alkyle en C₁ à C₆)carbonyle ; aminocarbonyle dans lequel le groupe amino est facultativement substitué par un groupe (alkoxy en C₁ à C₆) carbonyle, (alkyle en C₁ à C₆)carbonyle, (alcényle en C₂ à C₆)oxycarbonyle, (alcényle en C₂ à C₆) carbonyle, alkyle en C₁ à C₆ ou alcényle en C₂ à C₆ et facultativement encore substitué par un groupe alkyle en C₁ à C₆ ou alcényle en C₂ à C₆ ;
deux groupes R¹⁴ ou un groupe R¹³ et un groupe R¹⁴ dans X¹, X² et X³, conjointement avec les atomes auxquels ils sont attachés et, si cela est approprié, le groupe intermédiaire X², forment un cycle carbocyclique ou hétérocyclique pentagonal ou hexagonal et les groupes R¹³, R¹⁴ et R¹⁵ restants sont tels que définis ci-dessus ; ou bien
deux groupes R¹⁴ ou un groupe R¹³ et un groupe R¹⁴ présents sur des atomes adjacents représentent ensemble une liaison et les groupes R¹³, R¹⁴ et R¹⁵ restants sont tels que définis ci-dessus ;
X⁴ est un groupe phényle ou un hétérocycle aromatique monocyclique penta- ou hexagonal lié par C ou N contenant jusqu'à quatre hétéroatomes choisis parmi O, S et N et facultativement substitué sur C par au plus trois groupes choisis parmi les groupes alkylthio en C₁ à C₄ ; halogéno ; carboxy (alkyle en C₁ à C₄) ; halogéno (alkoxy en C₁ à C₄) ; halogéno (alkyle en C₁ à C₄) ; alkyle en C₁ à C₄ ; (alcényle en C₂ à C₄) ; (alkoxy en C₁ à C₄)carbonyle ; formyle ; (alkyle en C₁ à C₄)carbonyle ; (alcényle en C₂ à C₄)oxycarbonyle ; (alcényle en C₂ à C₄)carbonyle ; (alkyle en C₁ à C₄)carbonyloxy ; (alkoxy en C₁ à C₄)carbonyl(alkyle en C₁ à C₄) ; hydroxy ; hydroxy(alkyle en C₁ à C₄) ; mercapto (alkyle en C₁ à C₄) ; alkoxy en C₁ à C₄ ; nitro ; cyano ; carboxy ; amino ou aminocarbonyle facultativement substitué comme pour les substituants correspondants dans R³ ; (alkyle en C₁ à C₄)sulfonyle ; (alcényle en C₂ à C₄)-sulfonyle ; ou aminosulfonyle dans lequel le groupe amino est facultativement substitué par un groupe alkyle en C₁ à C₄ ou alcényle en C₂ à C₄ ; aryle ; aryl (alkyle en C₁ à C₄) ou aryl(alkoxy en C₁ à C₄) ; et facultativement substitué sur N par un groupe trifluorométhyle ; alkyle en C₁ à C₄ facultativement substitué par un groupe hydroxy, alkoxy en C₁ à C₆, (alkyle en C₁ à C₆) thio, halogéno ou trifluorométhyle ; alcényle en C₂ à C₄ ; aryle ; aryl(alkyle en C₁ à C₄) ; (alkoxy en C₁ à C₄)carbonyle ; (alkyle en C₁ à C₄)carbonyle ; formyle ; (alkyle en C₁ à C₆)sulfonyle ; ou aminocarbonyle dans lequel le groupe amino est facultativement substitué par un groupe (alkoxy en C₁ à C₄) carbonyle, (alkyle en C₁ à C₄)carbonyle, (alcényle en C₂ à C₄)oxycarbonyle, (alcényle en C₂ à C₄)carbonyle, alkyle en C₁ à C₄ ou alcényle en C₂ à C₄ et facultativement encore substitué par un groupe alkyle en C₁ à C₄ ou alcényle en C₂ à C₄ ;
à condition que lorsque X⁴ est un groupe phényle facultativement substitué, -V-X¹-X²-X³- ne soit pas - (CH₂) ₃O-, -CH₂-(alcényle en C₃) - ou - (CH₂) ₃CO-;
n est 0 et AB est NR¹¹CO, CO-CR⁸R⁹, CR⁶R⁷-CO, NR¹¹SO₂, CR⁶R⁷-SO₂ ou CR⁶R⁷-CR⁸R⁹, à condition que R⁸ et R⁹ ne soient pas un groupe hydroxy ou amino facultativement substitué et que R⁶ et R⁸ ne représentent pas une liaison ;
ou bien n est 1 et AB est NR¹¹CO, CO-CR⁸R⁹, CR⁶R⁷-CO, NR¹¹SO₂, CONR¹¹, CR⁶R⁷-CR⁸R⁹, O-CR⁸R⁹ ou NR¹¹-CR⁸R⁹ ;
et où :
chacun de R⁶ et R⁷, R⁸ et R⁹ est choisi indépendamment parmi : H ; un groupe alkoxy en C₁ à C₆ ; (alkyle en C₁ à C₆)thio ; halogéno ; trifluorométhyle ; azido ; alkyle en C₁ à C₆ ; alcényle en C₂ à C₆ ; (alkoxy en C₁ à C₆)carbonyle ; (alkyle en C₁ à C₆)carbonyle ; (alcényle en C₂ à C₆)oxycarbonyle ; (alcényle en C₂ à C₆) carbonyle ; hydroxy, amino ou aminocarbonyle facultativement substitué comme pour les substituants correspondants dans R³ ; (alkyle en C₁ à C₆) sulfonyle ; (alcényle en C₂ à C₆)sulfonyle ; ou aminosulfonyle en C₁ à C₆ dans lequel le groupe amino est facultativement substitué par un groupe alkyle en C₁ à C₆ ou alcényle en C₂ à C₆ ;
ou bien R⁶ et R⁸ représentent ensemble une liaison et R⁷ et R⁹ sont tels que définis ci-dessus ;
et chaque R¹¹ est indépendamment H ; un groupe trifluorométhyle ; alkyle en C₁ à C₆ ; alcényle en C₂ à C₆ ; (alkoxy en C₁ à C₆) carbonyle ; (alkyle en C₁ à C₆) - carbonyle ; ou aminocarbonyle dans lequel le groupe amino est facultativement substitué par un groupe (alkoxy en C₁ à C₆) carbonyle, (alkyle en C₁ à C₆) carbonyle, (alcényle en C₂ à C₆)oxycarbonyle, (alcényle en C₂ à C₆) carbonyle, alkyle en C₁ à C₆ ou alcényle en C₂ à C₆ et facultativement encore substitué par un groupe alkyle en C₁ à C₆ ou alcényle en C₂ à C₆ ;
ou bien, lorsque l'un de R³ et R⁶, R⁷, R⁸ ou R⁹ contient un groupe carboxy et l'autre contient un groupe hydroxy ou amino, ils peuvent former ensemble une liaison ester ou amide cyclique.

2. Composé selon la revendication 1, dans lequel Z⁵ est CH ou N, Z³ est CH ou CF et Z¹, Z² et Z⁴ sont chacun CH, ou bien Z¹ est N, Z³ est CH ou CF et Z², Z⁴ et Z⁵ sont chacun CH.

3. Composé selon l'une quelconque des revendications précédentes, dans lequel R¹ est un groupe méthoxy et R^{1a} est H ou, lorsque Z³ est CR^{1a}, il peut être C-F.

4. Composé selon l'une quelconque des revendications précédentes, dans lequel R³ est l'hydrogène ; un groupe CONH₂ ; 1-hydroxyalkyle ; CH₂CO₂H ; CH₂CONH₂ ; -CONHCH₂CONH₂ ; 1,2-dihydroxyalkyle ; CH₂CN ; 2-oxo-oxazolidine-5-yle ou 2-oxo-oxazolidine-5-yl(alkyle en C₁ à C₄).

5. Composé selon l'une quelconque des revendications précédentes, dans lequel n est 0 et soit A est CHOH et B est CH₂, soit A est NH et B est CO.

6. Composé selon l'une quelconque des revendications précédentes, dans lequel V est CH₂ et -X¹-X²-X³- est -(CH₂)₂-O-, -CH₂-CH=CH-, -(CH₂)₃-, -(CH₂)₂-NH-, -CH(OH)-CH₂-NH-, -CH₂NHCO-ou -CH₂CONH-.

7. Composé selon l'une quelconque des revendications précédentes, dans lequel X⁴ est un groupe 2-pyridyle, 3-fluorophényle, 3,5-difluorophényle ou 1,3-thiazole-2-yle.

8. Composé selon la revendication 1, choisi parmi les suivants :
(*R*)-1-(3,5-difluorophénylamino)-3-{4-[(*R*)-2-hydroxy-2-(6-méthoxyquinoléine-4-yl)éthyl]pipérazine-1-yl}propane-2-ol
(*R*/*S*)-1-(3,5-difluorophénylamino)-3-{4-[(*R*)-2-hydroxy-2-(6-méthoxyquinoléine-4-yl)éthyl]pipérazine-1-yl}propane-2-ol
3-(3,5-difluorophényl)-5-{4-[(*R*)-2-hydroxy-2-(6-méthoxyquinoléine-4-yl)éthyl]pipérazine-1-ylméthyl}-oxazolidine-2-one
*N*-(2-{4-[(*R*)-2-hydroxy-2-(6-méthoxyquinoléine-4-yl)éthyl]pipérazine-1-yl}éthyl)benzamide
3,5-difluoro-*N*-(2-{4-[(*R*)-2-hydroxy-2-(6-méthoxyquinoléine-4-yl)éthyl]pipérazine-1-yl}éthyl)benzamide
(2-{4-[(*R*)-2-hydroxy-2-(6-méthoxyquinoléine-4-yl)éthyl]pipérazine-1-yl}éthyl)amide d'acide pyridine-2-carboxylique
(2-{4-[(*R*)-2-hydroxy-2-(6-méthoxyquinoléine-4-yl)-éthyl]pipérazine-1-yl}éthyl)amide d'acide thiophène-2-carboxylique
(2-{4-[(R)-2-hydroxy-2-(6-méthoxyquinoléine-4-yl)-éthyl]pipérazine-1-yl}éthyl)amide d'acide furanne-2-carboxylique
3-cyano-*N*-(2-{4-[(*R*)-2-hydroxy-2-(6-méthoxyquinoléine-4-yl)éthyl]pipérazine-1-yl}éthyl)benzamide
*N*-(2-{4-[(*R*)-2-hydroxy-2-(6-méthoxyquinoléine-4-yl)éthyl]pipérazine-1-yl}éthyl)trifluorométhylbenzamide
(E)-1-{4-[(*R*)-2-hydroxy-2-(6-méthoxyquinoléine-4-yl)éthyl]pipérazine-1-yl}-4-phényl-but-3-ène-1-one
(*R*)-1-(6-méthoxyquinoléine-4-yl)-2-[4-(4-phénylbutyl)-pipérazine-1-yl]éthanol
(*S*)-1-(6-méthoxyquinoléine-4-yl)-2-[4-(4-phénylbutyl)-pipérazine-1-yl]éthylamine
*N*-(3,5-difluorophényl)-3-{4-[(*R*)-2-hydroxy-2-(6-méthoxyquinoléine-4-yl)éthyl]pipérazine-1-yl}propionamide
1-{4-[(*R*)-2-hydroxy-2-(6-méthoxyquinoléine-4-yl)-éthyl]pipérazine-1-yl}-3-phénoxypropane-2-ol
3-(3-fluorophényl)-5-{4-[(*R*)-2-hydroxy-2-(6-méthoxy-quinoléine-4-yl)éthyl]pipérazine-1-ylméthyl}oxazolidine-2-one et
(*S*)-1-{4-[(*R*)-2-hydroxy-2-(6-méthoxyquinoléine-4-yl)éthyl]pipérazine-1-yl}-3-phénylaminopropane-2-ol
ou un de leurs sels d'addition d'acide, sels d'ammonium quaternaire, produits de solvatation et/ou N-oxydes pharmaceutiquement acceptables.

9. Utilisation d'un composé selon la revendication 1, dans la fabrication d'un médicament destiné à être utilisé dans le traitement d'infections bactériennes chez des mammifères.

10. Composition pharmaceutique comprenant un composé selon la revendication 1 et un support pharmaceutiquement acceptable.

11. Procédé de préparation d'un composé selon la revendication 1, lequel procédé comprend la réaction d'un composé de formule (IV) avec un composé de formule (V) : 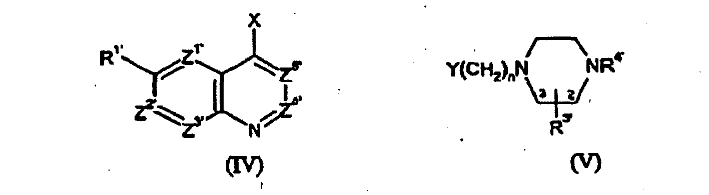 où n est tel que défini dans la formule (I) ; Z^{1'}, Z^{2'}, Z^{3'}, Z^{4'}, Z^{5'}, R^{1'}, R^{3'} et R^{4'} sont Z¹, Z², Z³, Z⁴, Z⁵, R¹, R³ et R⁴ tels que définis dans la formule (I) ou des groupes convertibles en ceux-ci ;
et X et Y peuvent être les combinaisons suivantes :
(i) X est A'-COW, Y est H et n est 0 ;
(ii) X est CR⁶=CR⁸R⁹, Y est H et n est 0 ;
(iii) X est un groupe oxiranne, Y est H et n est 0 ;
(iv) X est N=C=O et Y est H et n est 0 ;
(v) l'un de X et Y est CO₂R^{y} et l'autre est CH₂CO₂R^{x} ;
(vi) X est CHR⁶R⁷ et Y est C(=O)R⁸ ;
(vii) X est CR⁷=PR^{z}₃ et Y est C(=O)R⁹ et n = 1 ;
(viii) X est C(=O)R⁷ et Y est CR⁹=PR^{z}₃ et n = 1 ;
(ix) Y est COW et X est NHR^{11'} ou NR^{11'}COW et n = 0 ou 1
ou, lorsque n = 1, X est COW et Y est NHR^{11'}, NCO ou NR^{11'}COW ;
(x) X est NHR^{11'} et Y est C(=O)R⁸ et n = 1 ;
(xi) X est NHR^{11'} et Y est CR⁸R⁹W et n = 1 ;
(xii) X est NR^{11'} COCH₂W ou NR^{11'}SO₂CH₂W et Y est H et n = 0 ;
(xiii) X est CR⁶R⁷SO₂W et Y est H et n = 0 ;
(xiv) X est W ou OH et Y est CH₂OH et n est 1 ;
(xv) X est NHR^{11'} et Y est SO₂W ou X est NR^{11'}SO₂W et Y est H, et n est 0 ;
où W est un groupe partant, par exemple halogéno ou imidazolyle ; R^{x} et R^{y} sont des groupes alkyle en C₁ à C₆ ; R^{z} est un groupe aryle ou alkyle en C₁ à C₆ ; A' et NR^{11'} sont A et NR¹¹ tels que définis dans la formule (I), ou des groupes convertibles en ceux-ci ; et le groupe oxiranne est : 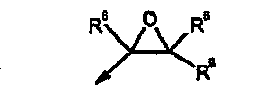 où R⁶, R⁸ et R⁹ sont tels que définis dans la formule (I) ;
puis, facultativement ou comme nécessaire, la conversion de A', Z^{1'}, Z^{2'}, Z^{3'} Z^{4'}, Z^{5'}, R^{1'}, R^{3'}, R^{4'} et NR^{11'} en A, Z¹, Z², Z³, Z⁴, Z⁵, R¹, R³, R⁴ et NR¹¹ ; la conversion de A-B en un autre A-B, l'interconversion de R¹, R³ et/ou R⁴ ; et/ou la formation d'un sel d'addition d'acide, d'un sel d'ammonium quaternaire, d'un produit de solvatation et/ou d'un N-oxyde pharmaceutiquement acceptable de ce composé.
